# EUROPEAN PATENT SPECIFICATION

(11) **EP 1 558 620 B1**
(45) Date of publication and mention of the grant of the patent: **10.09.2008**
(21) Application number: 03762788.2
(22) Date of filing: 04.07.2003
(51) Int. Cl.: C07F 15/00, A61K 31/00, A61P 35/00

(54) **RUTHENIUM ANTICANCER COMPLEXES**
RUTHENIUMKOMPLEXE MIT ANTIKREBSWIRKUNG
COMPLEXES DE RUTHENIUM ANTICANCEREUX

(30) Priority: 05.07.2002 GB 0215526
(43) Date of publication of application: 03.08.2005
(73) Proprietor: THE UNIVERSITY COURT OF THE UNIVERSITY OF EDINBURGH, Edinburgh EH8 9YL (GB)
(72) Inventor: SADLER, Peter, John, Penicuik EH26 8LW (GB); FERNANDEZ LAINEZ, Rafael, Edinburgh EH17 8HX (GB); HABTEMARIAM, Abraba, Edinburgh EH8 8AD (GB); MELCHART, Michael, Edinburgh EH8 9EW (GB); JODRELL, Duncan, Ian, Edinburgh EH12 8UB (GB)
(74) Representative: Watson, Robert James
(86) International application number: PCT/GB2003/002879
(87) International publication number: WO 2004/005304

(56) References cited:
- EP-A- 0 916 637
- SHIN, RICHARD Y. C. ET AL: "Arene-Ruthenium Complexes of an Acyclic Thiolate-Thioether and Tridentate Thioether Derivatives Resulting from Ring-Closure Reactions" INORGANIC CHEMISTRY (2003), 42(1), 96-106 , XP002259899
- BEN AMMAR, HAMED ET AL: "Synthesis of bis-oxazoline-ruthenium(II)-arene complexes. Combined catalytic isomerization and Claisen rearrangement of bis-allyl ether" JOURNAL OF ORGANOMETALLIC CHEMISTRY (2002), 662(1-2), 63-69 , XP002259901
- CHEN HAIMEI ET AL: "Organometallic ruthenium(II) diamine anticancer complexes: arene-nucleobase stacking and stereospecific hydrogen-bonding in guanine adducts." JOURNAL OF THE AMERICAN CHEMICAL SOCIETY. UNITED STATES 27 MAR 2002, vol. 124, no. 12, 27 March 2002 (2002-03-27), pages 3064-3082, XP002259900 ISSN: 0002-7863 cited in the application
- DATABASE CA [Online] CHEMICAL ABSTRACTS SERVICE, COLUMBUS, OHIO, US; BELL, MICHAEL N. ET AL: "Carbocyclic complexes incorporating macrocyclic ligands. The synthesis and single crystal x-ray structure of the binuclear species dichlorobis(.eta.-pentamethylcyclopentadie nyl)(1,4,7,10,13,1 6-hexathiacyclooctadecane)dirhodium bis(tetraphenylborate)" retrieved from STN Database accession no. 106:84806 XP002259903 & JOURNAL OF THE CHEMICAL SOCIETY, CHEMICAL COMMUNICATIONS (1986), (6), 471-2 ,
- DATABASE CA [Online] CHEMICAL ABSTRACTS SERVICE, COLUMBUS, OHIO, US; BENNETT, MARTIN A. ET AL: "Mono- and bis-(acetylacetonato) complexes of arene-ruthenium(II) and arene-osmium(II): variation of the binding mode of.eta.1-acetylacetonate with the nature of the arene" retrieved from STN Database accession no. 135:371841 XP002259904 & CANADIAN JOURNAL OF CHEMISTRY (2001), 79(5/6), 655-669 ,
- DATABASE CA [Online] CHEMICAL ABSTRACTS SERVICE, COLUMBUS, OHIO, US; DAVIES, DAVID L. ET AL: "(Arene)ruthenium Complexes with Bis(oxazolines): Synthesis and Applications as Asymmetric Catalysts for Diels-Alder Reactions" retrieved from STN Database accession no. 135:152944 XP002259905 & ORGANOMETALLICS (2001), 20(14), 3029-3034 ,
- DATABASE CA [Online] CHEMICAL ABSTRACTS SERVICE, COLUMBUS, OHIO, US; OHNISHI, TAKAFUMI ET AL: "Coordination behavior of ruthenium(II) complexes with alcohol ligand tethered to.eta.6-arene donor" retrieved from STN Database accession no. 131:257682 XP002259906 & CHEMISTRY LETTERS (1999), (8), 809-810 ,
- DATABASE CA [Online] CHEMICAL ABSTRACTS SERVICE, COLUMBUS, OHIO, US; EVERAERE, KATHELYNE ET AL: "(.beta.-Amino alcohol)(arene)ruthenium(II)-catalyzed asymmetric transfer hydrogenation of functionalized ketones - scope, isolation of the catalytic intermediates, and deactivation processes" retrieved from STN Database accession no. 134:295540 XP002259907 & EUROPEAN JOURNAL OF ORGANIC CHEMISTRY (2001), (2), 275-291 ,
- DATABASE CA [Online] CHEMICAL ABSTRACTS SERVICE, COLUMBUS, OHIO, US; WOISETSCHLAGER, OLIVER E. ET AL: "Hydrocarbon-bridged metal complexes. Part 49. Coordination chemistry of bis(ferrocenyl)-substituted 1,3-diketonates with ruthenium, rhodium, iridium, and palladium" retrieved from STN Database accession no. 132:308480 XP002259908 & ZEITSCHRIFT FUER ANORGANISCHE UND ALLGEMEINE CHEMIE (2000), 626(3), 766-774 ,
- DATABASE CA [Online] CHEMICAL ABSTRACTS SERVICE, COLUMBUS, OHIO, US; KATHO, AGNES ET AL: "Enantioselective hydride transfer hydrogenation of ketones catalyzed by [(.eta.6-p-cymene)Ru(amino acidato)Cl] and [(.eta.6-p- cymene)Ru(amino acidato)]3(BF4)3 complexes" retrieved from STN Database accession no. 132:222637 XP002259909 & JOURNAL OF ORGANOMETALLIC CHEMISTRY (2000), 593-594, 299-306 ,
- DATABASE CA [Online] CHEMICAL ABSTRACTS SERVICE, COLUMBUS, OHIO, US; MIYAKI, Y. ET AL: "Synthesis and reaction of ruthenium(II) complexes containing heteroatom donor (O, N, and P) tethered to.eta.6-arene ring" retrieved from STN Database accession no. 133:135395 XP002259910 & INORGANICA CHIMICA ACTA (2000), 300-302, 369-377 ,
- FALLER, J. W. ET AL: "Highly enantioselective Diels-Alder catalysis with a chiral ruthenium bisoxazoline complex" JOURNAL OF ORGANOMETALLIC CHEMISTRY (2001), 630(1), 17-22 , XP002259902
- DATABASE CA [Online] CHEMICAL ABSTRACTS SERVICE, COLUMBUS, OHIO, US; SIMAL, FRANCOIS ET AL: "Ruthenium complexes containing diamine-based ligands as catalysts for insertion of carbenes into O-H bonds of alcohols" retrieved from STN Database accession no. 130:222808 XP002259911 & TETRAHEDRON LETTERS (1999), 40(1), 63-66 ,
- DATABASE CA [Online] CHEMICAL ABSTRACTS SERVICE, COLUMBUS, OHIO, US; KUROSAWA, HIDEO ET AL: "Second sphere coordination behavior of aquo and amine ligands bound to a.eta.6-benzeneruthenium(II) cation" retrieved from STN Database accession no. 128:257553 XP002259912 & INORGANICA CHIMICA ACTA (1998), 270(1,2), 87-94 ,
- DATABASE CA [Online] CHEMICAL ABSTRACTS SERVICE, COLUMBUS, OHIO, US; KUHLWEIN, FRANK ET AL: "Metal complexes of dyes. Part 9. Transition metal complexes of curcumin and derivatives" retrieved from STN Database accession no. 127:228818 XP002259913 & ZEITSCHRIFT FUER ANORGANISCHE UND ALLGEMEINE CHEMIE (1997), 623(8), 1211-1219 ,
- DATABASE CA [Online] CHEMICAL ABSTRACTS SERVICE, COLUMBUS, OHIO, US; KRAEMER, ROLAND ET AL: "Metal complexes of biologically important ligands. LIII. Chiral half-sandwich complexes of rhodium(III), iridium(III), iridium(I), and ruthenium(II) with.alpha.-amino acid anions" retrieved from STN Database accession no. 112:198744 XP002259914 & CHEMISCHE BERICHTE (1990), 123(4), 767-78 ,
- DATABASE CA [Online] CHEMICAL ABSTRACTS SERVICE, COLUMBUS, OHIO, US; SHELDRICK, W. S. ET AL: "Synthesis and structural characterization of.eta.6- areneruthenium(II) complexes of.alpha.-amino acids with coordinating side chains" retrieved from STN Database accession no. 113:59793 XP002259915 & JOURNAL OF ORGANOMETALLIC CHEMISTRY (1989), 377(2-3), 357-66 ,
- DATABASE CA [Online] CHEMICAL ABSTRACTS SERVICE, COLUMBUS, OHIO, US; GOETZE, H. J. ET AL: "Separation of amino-acidato ruthenium(II) complexes by ion-pair chromatography" retrieved from STN Database accession no. 120:94149 XP002259916 & FRESENIUS' JOURNAL OF ANALYTICAL CHEMISTRY (1993), 346(6-9), 634-8 ,
- DATABASE CA [Online] CHEMICAL ABSTRACTS SERVICE, COLUMBUS, OHIO, US; CARMONA, DANIEL ET AL: "Heterobi- and Heterotetranuclear RuRh and RuIr Complexes with 2,2'-Biimidazolate and 2,2'-Bibenzimidazolate Anions as Bridging Ligands" retrieved from STN Database accession no. 122:214225 XP002259931 & ORGANOMETALLICS (1995), 14(4), 2066-80 ,
- STERN C ET AL: "The use of macrocyclic and polydentate ligands in ruthenium organometallic chemistry" JOURNAL OF ORGANOMETALLIC CHEMISTRY, ELSEVIER-SEQUOIA S.A. LAUSANNE, CH, vol. 593-594, January 2000 (2000-01), pages 86-95, XP004185686 ISSN: 0022-328X

## Description

This invention relates to ruthenium(II) compounds, to their use in medicine, particularly for the treatment and/or prevention of cancer, and to a process for their preparation.

Certain ruthenium(II) complexes have been proposed for use in treating cancer. For example, US 4980473 discloses 1,10-phenanthroline complexes of ruthenium(II) and cobalt(III) which are said to be useful for the treatment of tumour cells in a subject.

Some other ruthenium(II) and ruthenium(III) complexes which have been shown to exhibit antitumour activity are mentioned in Guo et al, Inorganica Chimica Acta, 273 (1998), 1-7, specifically *trans*-[RuCl₂(DMSO)₄], *trans-*[RuCl₄(imidazole)₂]⁻ and *trans*-[RuCl₄(indazole)₂]⁻. Clarke *et al* have reviewed the anticancer, and in particular the antimetastatic, activity of ruthenium complexes: Clem. Rev., 1999, 99, 2511-2533. Also, Sava has reviewed the antimetastatic activity in "Metal Compounds in Cancer Therapy" Ed by S P Fricker, Chapman and Hall, London 1994, p. 65-91.

Dale et al, Anti-Cancer Drug Design, (1992), 7, 3-14, describes a metronidazole complex of ruthenium(II) ie, [(η⁶-C₆H₆)RuCl₂(metronidazole)] and its effect on DNA and on *E. coli* growth rates. Metronidazole sensitises hypoxic tumour cells to radiation and appears to be an essential element of the complexes of Dale *et al*. There is no indication in Dale *et al* that the complexes would be at all effective in the absence of the metronidazole ligand.

Krämer et al, Chem Eur J., 1996, 2, No. 12, p. 1518-1526 discloses half sandwich complexes of ruthenium with amino esters.

Bennett et al, Canadian Journal of Chemistry, (2001), 79, 655-669 discloses certain ruthenium(II) complexes with acetylacetonate ligands.

Oro et al, J Chem Soc, Dalton Trans, (1990), 1463 describes ruthenium(II) complexes containing η⁶-p-cymene and acetylacetonate ligands.

WO 01/30790 discloses ruthenium(II) compounds and their use as anticancer agents. The compounds have neutral N-donor ligands and the resulting ruthenium complex is generally positively charged.

WO 02/02572 also discloses ruthenium(II) compounds that have activity against cancer cell lines. Again, the complexes are generally positively-charged. Complexes are disclosed containing a bidentate ligand which is a neutral diamine ligand.

Chen et al, J. Am. Chem. Soc., volume 124, no 12, 3064, (2002), describes the mechanism of binding of ruthenium complexes to guanine bases. The binding model requires NH bonds from a diamino ligand to be present in the complex for hydrogen bonding to the guanine base. Similarly, Morris et al, J. Med. Chem., volume 44, 3616-3621, (2001), describes the selectivity of ruthenium(II) complexes for binding to guanine bases.

There exists a need for novel anti-cancer compounds which can be used as alternatives to the compounds which are currently available.

Furthermore, there exists a need for compounds which are capable of binding to different DNA bases. Binding to different DNA bases can provide a compound that exhibits increased activity to drug-resistant tumour cells.

The present invention provides a novel class of ruthenium(II) complexes having anti-tumour activity.

According to the present invention, there is provided a ruthenium(II) compound of formula (I): 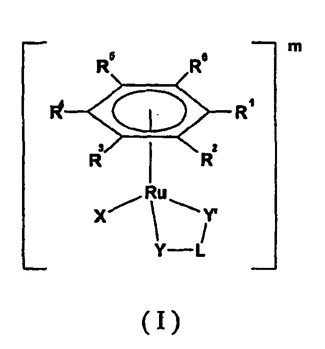 wherein: R¹, R², R³, R⁴, R⁵ and R⁶ independently represent H, (C₁-C₆)alkyl, (C₂-C₆)alkenyl, (C₂-C₆)alkynyl, hydroxy(C₁-C₆)alkyl, amino(C₁-C₆)alkyl, halo, CO₂R⁷, CONR⁸R⁹, COR¹⁰, SO₃H, SO₂N R¹¹R¹², aryloxy, (C₁-C₆)alkoxy, (C₁-C₆)alkylthio, -N=N-R¹³, NR¹⁴R¹⁵, aryl or aralkyl, which latter two groups are optionally substituted on the aromatic ring by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7a}, CONR^{8a}R^{9a}, COR^{10a}, SO₃G, SO₂NR^{11a}R^{12a}, aryloxy, (C₁-C₆)alkoxy, (C₁-C₆)alkylthio, -N=N-R^{13a}, NR^{14a}R^{15a} , or R¹ and R² together with the ring to which they are bound represent a saturated or unsaturated carbocyclic or heterocyclic group containing up to three 3- to 8- membered carbocyclic or heterocyclic rings, wherein each carbocyclic or heterocyclic ring may be fused to one or more other carbocyclic or heterocyclic rings, and wherein each of the rings may be optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R^{15b} or (C₁-C₆)alkoxy;
R⁷, R⁸, R⁹, R¹⁰, R¹¹, R¹², R¹³, R¹⁴, R¹⁵, R^{7a}, R^{8a}, R^{9a}, R^{10a}, R^{11a}, R^{12a}, R^{13a}, R^{14a}, R^{15a}, R^{7b}, R^{8b}, R^{9b}, R^{10b}, R^{11b}, R^{12b}, R^{13b}, R^{14b} and R^{15b} are independently selected from H, (C₁-C₆)alkyl, aryl or aralkyl;
X is a neutral or negatively charged 0-, N- or S- donor ligand or halo;
G and G' are independently selected from alkali metals (eg, sodium or potassium), aryl, aralkyl and (C₁-C₆) alkyl;
Y-L-Y' is a bidentate ligand bearing negative charge with a proportion of the charge on both Y and Y', Y and Y' are independently selected from O, S or NR¹⁶, wherein R¹⁶ is H, (C₁-C₆)alkyl, aryl or aralkyl, and L is a group linking Y and Y' and comprises one or more groups selected from (C₁-C₆)alkylene, (C₁-C₆)alkenylene, (C₁-C₆)alkynylene, arylene, aralkylene, alkarylene, each of said latter six groups being optionally substituted, ferrocenylene, Se, Se-Se, S-S, N=N and C=O;
m is -1, 0 or +1 and the compound comprises a counterion when m is -1 or +1; the compound of formula (I) optionally being in the form of a dimer in which two L groups are linked either directly or through a group comprising one or more of (C₁-C₆)alkylene, (C₁-C₆)alkenylene, arylene, aralkylene, alkarylene, Se, Se-Se, S-S, N=N and C=O or in which L bears two Y groups and two Y' groups;
with the proviso that:
when Y-L-Y' is (CH₃C(O)CHC(O)CH₃)⁻, X is halo or an N-donor ligand, R¹, R², R³, R⁴, R⁵ and R⁶ together with the ring to which they are bound do not represent 4-isopropyl-1-methylbenzene;
when Y-L-Y' is (CH₃C(O)CHC(O)CH₃)⁻ and X is chloro, (CH₃)₂SO, CH₃CN, pyridine or (CH₃C(O)CHC(O)CH₃)⁻: R¹, R², R³, R⁴, R⁵ and R⁶ are not all H or all methyl; R¹, R³ and R⁵ are not all H when R², R⁴ and R⁶ are all methyl; and R², R⁴ and R⁶ are not all H when R¹, R³ and R⁵ are all methyl; and
when Y-L-Y' is (CF₃C(O)CHC(O)CF₃)⁻ and X is chloro, R¹, R², R³, R⁴, R⁵ and R⁶ are not all H or all methyl; R¹, R³ and R⁵ are not all H when R², R⁴ and R⁶ are all methyl; and R², R⁴ and R⁶ are not all H when R¹, R³ and R⁵ are all methyl;
when Y-L-Y' is (CF₃C(O)CHC(O)OEt)⁻ and X is chloro, R¹, R², R³, R⁴, R⁵ and R⁶ together with the ring to which they are bound do not represent 4-isopropyl-1-methylbenzene;
when Y-L-Y' is ((ferrocenylene)C(O)CHC(O)(ferrocenylene))⁻ and X is chloro, R¹, R², R³, R⁴, R⁵ and R⁶ together with the ring to which they are bound do not represent 4-isopropyl-1-methylbenzene;
when R¹, R², R³, R⁴, R⁵ and R⁶ together with the ring to which they are bound represent 4-isopropyl-1-methylbenzene and X is chloro, Y-L-Y' is neither (3-OCH₃-4-OH-PhCHCHC(O)CHC(O)CHCH-3-OCH₃-4-OH-Ph)⁻, (3-OCH₃-4-OCH₃-PhCHCHC(O)C(CH₃)C(O)CHCH-3-OCH₃-4-OCH₃-Ph)⁻, (3-OCH₃-4-COOCH₃-PhCHCHC(O)CHC(O)CHCH-3-OCH₃-4-COOCH₃-Ph)⁻, (4-OH-PhCHCHC(O)CHC(O)CHCH-4-OH-Ph)⁻ nor (3-OCH₃-4-OCH₃-PhCHCHC(O)CHC(O)CHCH-3-OCH₃-4-OCH₃-Ph)⁻.

The compounds of the invention may be in the form of pharmaceutically acceptable salts, solvates and/or prodrugs. Prodrugs are variants of the compounds of the invention which can be converted to compounds of formula (I) *in vivo*.

The compounds of formula (I) may have one or more chiral centres. When the compounds of formula (I) have one or more chiral centres, they may be in the form of one enantiomer, may be enriched in one enantiomer or may be a racemic mixture.

The term "alkyl" as used herein includes C₁ to C₆ alkyl groups which may be branched or unbranched and may be open chain or, when they are C₃ to C₆ groups, cyclic. Unbranched open chain alkyl groups include, for example, methyl, ethyl, propyl, butyl, pentyl and hexyl. Branched open chain alkyl groups include, for example, 2-propyl, 2-butyl and 2-(2-methyl)propyl. Cyclic groups include cyclopropyl, cyclobutyl, cyclopentyl and cyclohexyl. The alkyl groups in the compounds of the invention may optionally be substituted. Substituents include one or more further unsubstituted alkyl groups and/or one or more further substituents, such as, for example, cyano, nitro, -CO₂(C₁-C₆)alkyl, halo, thiol (SH), thioether (eg, S-(C₁-C₆)akyl) and sulfonate. The term "alkoxy" means -0-alkyl. The term "alkylthio" means -S-alkyl.

The terms "hydroxy(C₁-C₆)alkyl" and "amino(C₁-C₆)alkyl" refer to alkyl groups, as defined above, substituted with one or more hydroxyl (OH) or amino (NH₂) groups, respectively.

The terms "alkenyl" and "alkynyl" are defined similarly to the term "alkyl" but refer to groups that contain from 2 to 6 carbon atoms and include one or more carbon-carbon double bonds or one or more carbon-carbon triple bonds, respectively. Alkenyl and alkynyl groups may be optionally substituted in the same way as alkyl groups. Examples of alkenyl groups are ethenyl, 1-propenyl, 2-propenyl, 1-butenyl, 2-butenyl, 1,4- butadienyl, cyclohexenyl and cyclohexadienyl.

The term "alkylene" is defined similarly to the definition of the term "alkyl" but includes C₂ to C₆ groups and represents a divalent species with radicals separated by two or more (eg, from two to six) carbon atoms linked in a chain. Preferably, the alkylene groups are straight chain groups. Alkylene groups are optionally substituted in the alkylene chain, preferably with one or more phenylene (eg, 1-4-phenylene) and/or -CONR^{1x}- groups and/or -NR^{2x}- groups, where R^{1x} and R^{2x} independently represent H, alkyl, aryl or aralkyl. Preferably, R^{1x} and R^{2x} are H or C₁ to C₃ alkyl. The terms "alkenylene" and "alkynylene" are defined similarly and refer to divalent radicals containing one or more carbon-carbon double bonds or one or more carbon-carbon triple bonds, respectively.

The term "aryl" as used herein includes aromatic carbocyclic rings such as phenyl, naphthyl and anthracenyl and heterocyclic rings such as pyridyl, imidazolyl, pyrrolyl and furanyl. Aryl groups may optionally be substituted with one or more substituents including, for example, (C₁-C₆)alkyl, cyano, nitro, hydroxyl, halo(C₁-C₆)alkyl, -CO₂(C₁-C₆)alkyl, halo, thiol (SH), thioether (eg, S-(C₁-C₆)alkyl) and sulfonate (SO₃H). The term "aryloxy" means -O-aryl.

The term "heterocyclic ring" refers to a 3-, 4-, 5-, 6-, -7, or 8- (preferably 5-, 6-or 7-) membered saturated or unsaturated ring, which may be aromatic or nonaromatic, containing from one to three heteroatoms independently selected from N,O and S, eg, indole.

The term "arylene" refers to a divalent radical comprising an aromatic carbocyclic or heterocyclic ring in which the radicals are present at different positions on the ring. An example of an arylene group is 1,4-phenylene.

The term "aralkyl" means alkyl substituted with aryl eg, benzyl. The term "alkaryl" means aryl substituted with alkyl eg, methylphenyl.

The term "aralkylene" refers to a divalent radical that can be derived from an aralkyl group eg, 1-methylene-4-phenyl. Each of the two radicals may be present on the aryl ring or on the alkyl group or one of the radicals may be present on the alkyl group and the other radical present on the aryl ring. The term "alkarylene" is defined similarly.

The term ferrocenylene refers to a diradical derived from ferrocene (FeCp₂). Each radical may be present on the same ring or on different rings.

The term "halo" means a halogen radical selected from fluoro, chloro, bromo and iodo. Chloro is particularly preferred. When X is halo in formula (I), it will be appreciated that X may be thought of as having at least some of the character of a negatively charged ion rather than being covalently bonded to the ruthenium atom. Indeed, all ligands X may have some ionic as well as some covalent character.

The term "haloalkyl" means alkyl substituted with one or more halo groups eg, trifluoromethyl.

In the compounds of the invention, R¹ and R² together with the ring to which they are bound in compounds of formula (I) may represent an *ortho*- or *peri*fused carbocyclic or heterocyclic ring system. R¹ and R² together with the ring to which they are bound may, for example, represent a wholly carbocyclic fused ring system such as a ring system containing 2 or 3 fused carbocyclic rings eg, optionally substituted, optionally hydrogenated naphthalene or anthracene. Thus, R¹ and R² together with the ring to which they are bound in compounds of formula (I) may represent a fused tricyclic ring such as anthracene or a mono, di, tri, tetra or higher hydrogenated derivative of anthracene. For example, R¹ and R² together with the ring to which they are bound in formula (I) may represent anthracene, 1, 4-dihydroanthracene or 1, 4, 9, 10-tetrahydroanthracene. Examples of R¹ and R² together with the ring to which they are bound in compounds of formula (I) representing heterocyclic ring systems include those compounds in which R¹ and R² together with the ring to which they are bound represent 2,3-benzofuran, indole or benzo[b]thiophene.

Preferably, R¹, R², R³, R⁴, R⁵ and R⁶ are independently selected from H, (C₁-C₆)alkyl and phenyl or R¹ and R² together with the ring to which they are bound represent anthracene or a hydrogenated derivative of anthracene, said phenyl and anthracene or a hydrogenated derivative of anthracene group being optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, phenyl, benzyl, halo, carboxyl, CO₂(C₁-C₆)alkyl, CONH₂, COH, CO(C₁-C₆)alkyl, SO₃H, SO₂NH₂, phenoxy, (C₁-C₆)alkylthio, NH₂ or (C₁-C₆)alkoxy_{.} Most preferably, one of R¹, R², R³, R⁴, R⁵ and R⁶ is phenyl and the other groups are all H, or one or two of R¹, R², R³, R⁴, R⁵ and R⁶ is or are (C₁-C₆)alkyl and the other groups are H, or R¹ and R² together with the ring to which they are bound represent anthracene or a hydrogenated derivative of anthracene.

It can be desirable for the compounds of the invention to be uncharged since this may assist the transport of the compounds in *is vivo* systems and thus their anticancer cytotoxic activity. Therefore, in formula (I), it is preferred that m is 0. When m is +1 or -1, the compounds of formula (I) comprise a counterion. Suitable counterions include non-nucleophilic ions such as, for example, PF₆⁻ and BF₄⁻.

In compounds of formula (I), X is a neutral or negatively charged O-, N- or S-donor ligand or halo. Suitable ligands include, for example, H₂O, di((C₁-C₆)alkyl)S(O), (C₁-C₆)alkylCO₂⁻ or di((C₁-C₆)alkyl)C=O. Other ligands include, for example, N-donor nitrile ligands (eg, compounds of formula (C₁-C₆)alkylCN) and N-donor pyridine ligands, optionally substituted at one or more of the carbon rings of the pyridine ring eg, by (C₁-C₆)alkyl or halo. Other suitable ligands are (C₁-C₆)alkyl primary amines such as methylamine and ethylamine. Preferably, X is halo or CH₃CN, most preferably, X is chloro.

Y-L-Y' is a bidentate ligand. The ligand has an overall negative charge ie, the ligand would bear a negative charge when not present in the complexes of the invention. The ligand may bear a single negative charge or may have more than one negative charge eg, by being a dianion. Preferably, Y-L-Y' has a single or double negative charge. The charge may be distributed throughout the ligand but a proportion of the charge is present on both Y and Y'. The charge may be due to the formation of an anion by deprotonation at Y and/or Y' or by delocalisation of the charge from Y to Y' or *vice versa*. Preferably, there is delocalisation of the charge from Y to Y' or from Y' to Y by conjugation of electrons through L. L may comprise one or more groups, for example L can comprise an alkylene group having a CO group at each end of the alkylene chain. Preferably, Y-L-Y' is selected from ligands of formulae 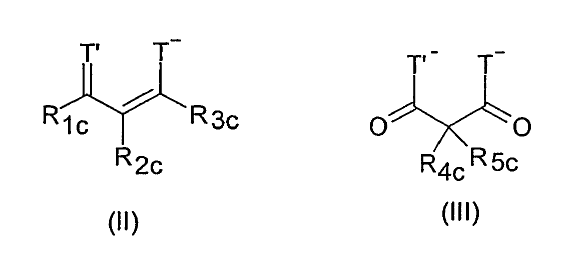 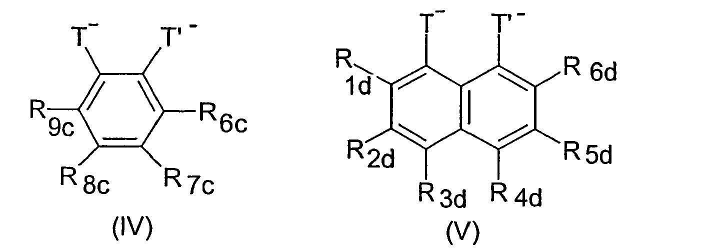 (II) to (X): 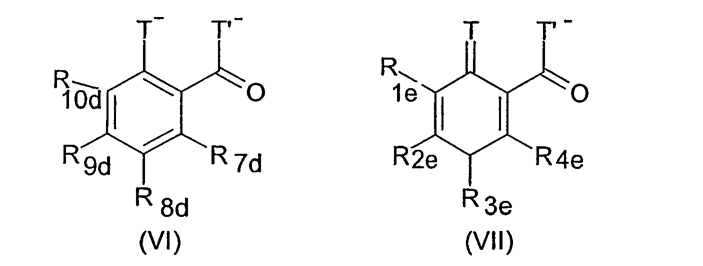 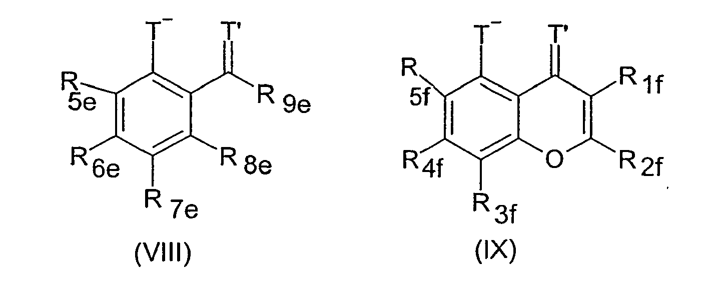 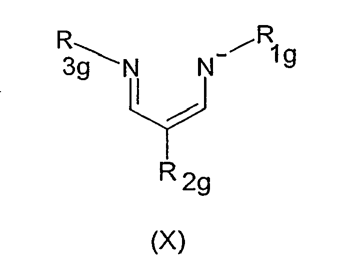 wherein T and T' are independently selected from O and S,
R_{1g} and R_{3g} are independently H, (C₁-C₆)alkyl, aryl or aralkyl,
R_{1c} to R_{5f} (i.e., R_{1c}, R_{2c}, R_{3c}, P_{4c}, R_{5c}, R_{6c}, R_{7c}, R_{8c}, R_{9c}, R_{1d}, R_{2d}, R_{3d}, R_{4d}, R_{5d}, R_{6d}, R_{7d}, R_{8d}, R_{9d}, R_{10d}, R₁ₑ, R₂ₑ, R₃ₑ, R₄ₑ, R₅ₑ, R₆ₑ, R₇ₑ, R₈ₑ, R₉ₑ, R_{1f}, R_{2f}, R_{3f}, R_{4f} and R_{5f}) and R_{2g} are independently H, (C₁-C₆)alkyl, aryl, aralkyl, wherein the latter two groups and the corresponding goups for R_{1g} and R_{3g} are optionally substituted by one or more groups independently selected from (C₁-C₆)akyl, (C₂-C₆)alkenyl, (C₂-C₆)alkynyl, hydroxy(C₁-C₆)alkyl, amino(C₁-C₆)alkyl, aryl, aralkyl, halo, carboxyl, CO₂R^{7b}, CONR^{8b}R^{9b}, COR^{10b}, SO₃G', SO₂N R^{11b}R^{12b}, aryloxy, (C₁-C₆)alkylthio, -N=N-R^{13b}, NR^{14b}R^{15b} or (C₁-C₆)alkyoxy, wherein R^{7b}, R^{8b}, R^{9b}, R^{10b}, R^{11b}, R^{12b}, R^{13b}, R^{14b} and R^{15b}are independently selected from H, (C₁-C₆)alkyl, aryl or aralkyl.

The compounds of formula (I) may be in the form of dimers, which may also be termed dinuclear complexes - such complexes contain two ruthenium atoms. Dinuclear complexes can be provided by employing a ligand which comprises two linked ligands Y-L-Y' so as to bridge between two ruthenium centres. Therefore, in another embodiment of the invention, Y-L-Y' may be selected from ligands of formulae (XI) to (XV): 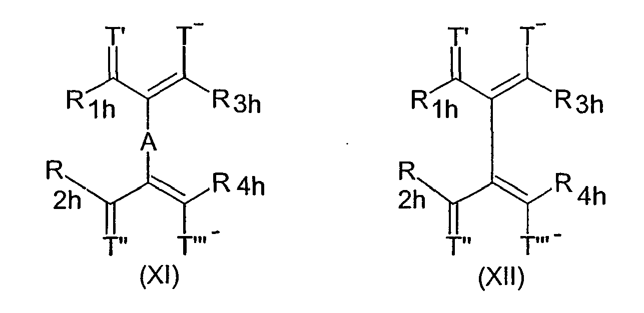 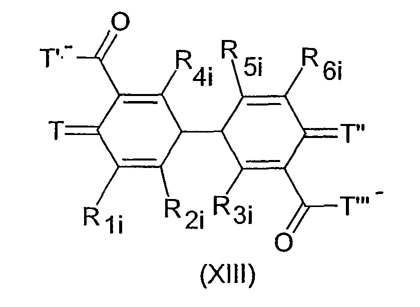 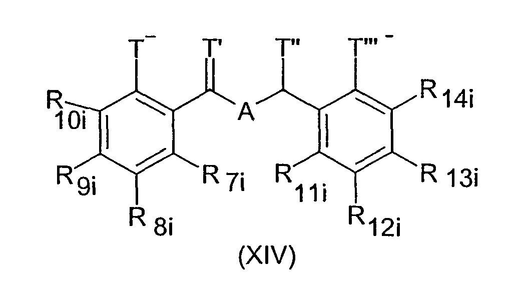 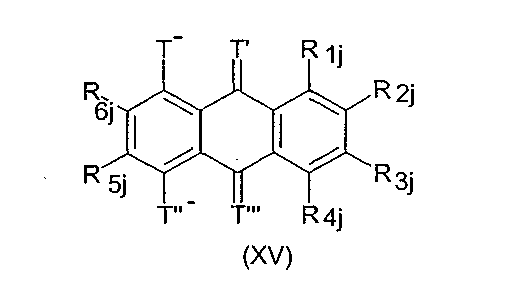 wherein T, T', T" and T''' are independently selected from O and S, A comprises one or more groups selected from (C₁-C₆)alkylene, (C₁-C₆)alkenylene, (C₁-C₆)alkynylene, arylene, aralkylene, alkarylene, ferrocenylene, Se, Se-Se, S-S, N=N and C=O,
and R₁ₕ to R₆ⱼ (i.e., R₁ₕ, R₂ₕ, R₃ₕ, R₄ₕ, R₁ᵢ, R₂ᵢ, R₃ᵢ, R₄ᵢ, R₅ᵢ, R₆ᵢ, R₇ᵢ, R₈ᵢ, R₉ᵢ, R₁₀ᵢ, R₁₁ᵢ, R₁₂ᵢ, R₁₃ᵢ, R₁₄ᵢ, R₁ⱼ, R₂ⱼ, R₃ⱼ, R₄ⱼ, R₅ⱼ, and R₆ⱼ) are independently H, (C₁-C₆)alkyl, aryl, aralkyl, wherein the latter two groups are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G, SO₂N R^{11b}R^{12b}, aryloxy, (C₁-C₆)alkylthio, -N=N-R^{13b}, NR^{14b}R^{15b} or (C₁-C₆)alkoxy, wherein R^{7b}, R^{8b}, R^{9b}, R^{10b}, R^{11b}, R^{12b}, R^{13b}, R^{14b} and R^{15b}are independently selected from H, (C₁-C₆)alkyl, aryl or aralkyl.

Particularly preferred compounds of formula (I) are those in which Y-L-Y' is: 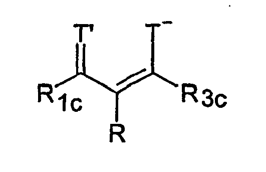 wherein T and T' are independently O and S, and
R, R_{1c} and R_{3c} are independently H, (C₁-C₆)alkyl, aryl, aralkyl, wherein the latter two groups are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, carboxyl, CO₂R^{7b}, CONR^{8b}R^{9b}, COR^{10b}, SO₃G³, SO₂N R^{11b}R^{12b}, aryloxy, (C₁-C₆)alkylthio, -N=N-R^{13b}, NR^{14b}R^{15b} or (C₁-C₆)alkoxy, wherein R^{7b}, R^{8b}, R^{9b}, R^{10b}, R^{11b}, R^{12b}, R^{13b}, R^{14b} and R^{15b}are as defined above. Even more preferably, T and T' are both O and R, R_{1c} and R_{3c} are independently (C₁-C₆)alkyl or phenyl, said phenyl optionally substituted by (C₁-C₆)alkyl, hydroxy(C₁-C₆)alkyl, amino(C₁-C₆)alkyl, halo, carboxyl, CO₂(C₁-C₆)alkyl, CONH₂, COH, CO(C₁-C₆)alkyl, SO₃H, SO₂NH₂, phenoxy, (C₁-C₆)alkylthio, NH₂ or (C₁-C₆)alkoxy. Most preferably, R is H or (C₁-C₆)alkyl and R_{1c} and R_{3c} are independently unsubstituted (C₁-C₆)alkyl or optionally substituted phenyl.

In the compounds of the invention, it is preferred that Y and Y' (including T, T', T" and T''' in the above formulae) are all O.

Compounds of formula (I) and ligands of formula Y-L-Y' may exist in one or more tautomeric forms, all of which are covered by the present invention. For example, ligands of formula: 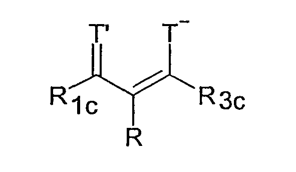 may have the following tautomeric forms:

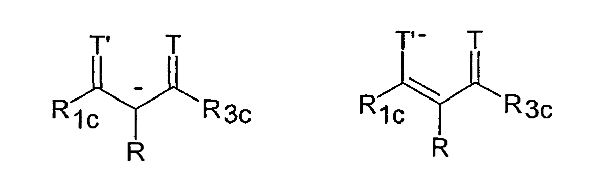

The existence and nature of the tautomers will depend on the structure of Y-L-Y'.

A particularly preferred group of compounds of formula (I) are those in which:
X is halo (preferably chloro);
R¹, R², R³, R⁴, R⁵ and R⁶ are independently selected from H, (C₁-C₆)alkyl and phenyl or R¹ and R² together with the ring to which they are bound represent anthracene or a hydrogenated derivative of anthracene, said phenyl and anthracene or a hydrogenated derivative of anthracene group being optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, phenyl, benzyl, halo, carboxyl, CO₂(C₁-C₆)alkyl, CONH₂, COH, CO(C₁-C₆)alkyl, SO₃H, SO₂NH₂, phenoxy, (C₁-C₆)alkylthio, NH₂ or (C₁-C₆)alkoxy; Y-L-Y' is: 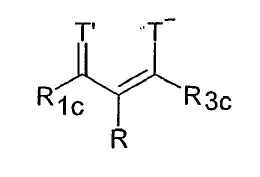
wherein T and T' are both O, R is H or (C₁-C₆)alkyl and R_{1c} and R_{3c} are independently (C₁-C₆)alkyl or phenyl, said phenyl optionally substituted by from one to three groups selected from (C₁-C₆)alkyl, hydroxy(C₁-C₆)alkyl, amino(C₁-C₆)alkyl, halo, carboxyl, CO₂(C₁-C₆)alkyl, CONH₂, COH, CO(C₁-C₆)alkyl, SO₃H, SO₂NH₂, phenoxy, (C₁-C₆)alkylthio, NH₂ or (C₁-C₆)alkoxy.

A further preferred group of compounds of formula (I) is that in which:
R¹, R², R³, R⁴, R⁵ and R⁶ are all H, or one of R¹, R², R³, R⁴, R⁵ and R⁶ is phenyl and the other groups are all H, or one or two of R¹, R², R³, R⁴, R⁵ and R⁶ is or are (C₁-C₆)alkyl and the other groups are all H, or R¹ and R² together with the ring to which they are bound represent anthracene or a hydrogenated derivative of anthracene;
X is chloro; and
Y-L-Y' is: 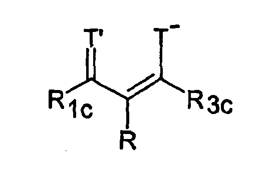
wherein T and T' are both O, R is H and R_{1c} and R_{3c} are independently (C₁-C₆)alkyl or R_{1c} and R_{3c} are independently unsubstituted (C₁-C₆)alkyl or optionally substituted phenyl.

The compounds of the invention have been found to exhibit cytotoxic activity against cancer cell lines and can therefore be expected to show anticancer activity. The compounds may be used to kill cells *in vivo* or *ex vivo*.

In another embodiment, therefore, the present invention provides a compound of formula (I) as defined above without the provisos for use in thereapy. The invention also contemplates the provision of a compound of formula (I) as defined above without the provisos for use in the treatment and/or prevention of cancer and the use of a compound of formula (I) as defined above without the provisos in the treatment and/or prevention of cancer.

Also provided by the invention is the use of a compound of formula (I) as defined above without the provisos in the manufacture of a medicament for the treatment and/or prevention of cancer.

Further provided by the invention is a pharmaceutical composition comprising a compound of formula (I) according as defined above without the provisos together with one or more pharmaceutically acceptable excipients.

Yet another aspect of the invention is a method of treating and/or preventing cancer which comprises administering to a subject a therapeutically effective amount of a compound of formula (I) as defined above without the provisos or a composition of the invention.

The compounds of the invention may be used directly against a tumour. Alternatively or additionally, the compounds may be used to prevent or inhibit metastasis and/or to kill secondary tumours. It will be understood that the prevention or inhibition of metastasis is encompassed by the term "preventing cancer", as used herein.

The term tumour includes all forms of neoplastic cell growth, including tumours of the lung, liver, blood cells, skin, pancreas, stomach, colon, prostate, uterus, breast, lymph glands and bladder. Ovarian tumours may be especially suitable for treatment according to the invention.

Compounds of the invention may be effective in treating and/or preventing tumours caused by cells that are resistant to other cytotoxic drugs, such as cisplatin, for example.

Certain compounds of the invention have the surprising advantage that they do not bind selectively to guanine bases but show roughly equal affinity for binding to guanine and adenine. This effect was unexpected, given that the presence of an N-H group available for hydrogen bonding was thought to be a requirement for bonding to guanine. Furthermore, binding to guanine and adenine gives the compounds a potentially greater ability to be less susceptible to drug resistance in tumour cells. In one embodiment, the compounds can be used in a method of binding non-selectively to guanine bases, preferably a method of binding to guanine and adenine bases with roughly equal affinity. This method may be, for example, a method of killing cells in vivo or ex vivo, or another method such as the separation of guanine and adenine bases from a mixture.

The invention also provides a pharmaceutical composition comprising one or more compounds of the invention together with one or more pharmaceutically acceptable excipients. Suitable excipients include diluents and/or carriers.

The compounds of the invention may be administered by a number of routes including, for example, orally, parenterally (eg, by injection intramuscularly, intravenously or subcutaneously), topically, nasally or via slow releasing microcarriers. Thus, suitable excipients for use in the pharmaceutical compositions of the invention include saline, sterile water, creams, ointments, solutions, gels, pastes, emulsions, lotions, oils, solid carriers and aerosols.

The compositions of the invention may be formulated in unit or sub-unit dosage form including, for example, tablets, capsules and lozenges and containers containing the composition in a form suitable for parenteral administration. Preferably, the compositions are in a form that is suitable for injection.

The specific dosage level of the compounds and compositions of the invention will depend upon a number of factors, including the biological activity of the specific compound used and the age, body weight and sex of the subject. It will be appreciated that the subject may be a human or a mammalian animal.

The compounds and compositions of the invention can be administered alone or in combination with other compounds. The other compounds may have a biological activity which complements the activity of the compounds of the invention eg, by enhancing its effect in killing tumours or by reducing any side-effects associated with the compounds of the invention.

In another embodiment, the present invention provides a process for preparing the compound of formula (I) which comprises the reaction of a compound of formula [(η⁶-C₆(R¹)(R²)(R³)(R⁴)(R⁵)(R⁶))RuX₂], optionally in the form of a dimer, with Y-L-Y', in a suitable solvent for the reaction, wherein R¹,R², R³, R⁴, R⁵, R⁶, X, Y,Y' and L are as defined for formula (I) above. Preferably, the process comprises the reaction of a compound of formula [(η⁶-C₆(R¹)(R²)(R³)(R⁴)(R⁵)(R⁶))RuX₂], optionally in the form of a dimer, with a salt comprising Y-L-Y' as an anion (eg, a salt of an alkali metal such as sodium) at a temperature of from 0°C to 100°C (eg, 10°C to 50 °C) in a polar solvent such as a di(C₁-C₆)alkyl ketone (eg, acetone) or water or mixtures thereof. The compound of formula (I) can be separated from the reaction mixture, for example by extraction into a less polar solvent (eg, dichloromethane) and, optionally, purified (eg, by recrystallisation from a suitable solvent or mixture of two or more different solvents).

Suitable compounds of formula [(η⁶-C₆(R¹)(R²)(R³)(R⁴)(R⁵)(R⁶))RuX₂] for use as starting materials (starting ruthenium complexes) in the process of the invention can be produced as described in WO 01/30790 and WO 02/02572. Compounds of formula Y-L-Y' are either commercially available or can be synthesised by routes well known to those skilled in the art.

The invention will now be described with reference to the following nonlimiting examples.

### Examples

### A. Synthesis

### Example 1

### [(η⁶-p-cymene)RuCl(H₃CCOCHCOCH₃ - O,O)].

The compound of Example 1 was prepared using the method described by D. Carmona et al, J. Chem. Soc., Dalton Trans., 1990, 1463 - 1476.

[(η⁶-*p*-cymene)RuCl₂]₂ (250 mg, 0.41 mmol) and sodium acetylacetonate monohydrate (150 mg, 1.07 mmol) were stirred in acetone (25 ml) at room temperature (RT) for 50 min. The extraction step then involved removal of the solvent *in vacuo* on a rotary evaporator, extraction of the product with dichloromethane, filtration using Whatman No. 541 filter paper, followed by removal of the solvent again on a rotary evaporator. The final product was recrystallised from acetone/diethyl ether and left in a freezer at 253 K overnight. The red crystals (180 mg, 0.48 mmol, 59% yield) were collected and dried *in vacuo*. ¹H NMR (CDCl₃): δ 5.43 (d, 2H), 5.18 (d, 2H), 5.13 (s, 1H), 2.85 (sp, 1H), 2.24 (s, 3H), 1.97 (s, 6H), 1.30 (d, 6H).

### Example 2

### [(η⁶-p-cymene)RuCl(F₃CCOCHCOCF₃ - O,O)]

[(η⁶-*p*-cymene)RuCl₂]₂ (250 mg, 0.41 mmol) and sodium hexafluoroacetylacetonate (260 mg, 1.13 mmol) were stirred in acetone (25 ml) at RT for 40 min. After the extraction step using dichloromethane (see Example 1), the final product was dissolved in diethyl ether, the solution was concentrated on a rotary evaporator, and hexane was added. The vessel was left in a freezer at 253K overnight. The crystals (277 mg, 0.58 mmol, 70.7% yield) were collected and dried *in vacuo*. ¹H NMR (CDCl₃): δ 5.86 (s, 1H), 5.63 (d, 2H), 5.36 (d, 2H), 2.88 (sp, 1H), 2.24 (s, 3H), 1.33 (d, 6H).

### Example 3

### [(η⁶-p-cymene)RuCl(C₆H₅COCHCOC₆H₅ - O,O)]

1,3-diphenyl-1,3-propanedione (277 mg, 1.24 mmol) and sodium methoxide (62 mg, 1.15 mmol) were stirred in methanol (30 ml) at room temperature (RT) for 100 min. The solvent was removed *in vacuo* and the product was washed with diethyl ether, which was then removed *in vacuo* on a rotary evaporator. This sodium salt (210 mg, 0.85 mmol, 73.9% yield) and [(η⁶-*p*-cymene)RuCl₂]₂ (240 mg, 0.39 mmol) were stirred in acetone (25 ml) at RT for 40 min. After the extraction step using dichloromethane (see Example 1), the final product was dissolved in acetone, concentrated on a rotary evaporator, and diethyl ether was added. The solution was left in a freezer at 253 K overnight. The crystals (238 mg, 0.48 mmol, 61.8% yield) were collected and dried *in vacuo*. ¹H NMR (CDCl₃): δ 7.88 (m, 2H), 7.43 (m, 2H), 7.36 (m, 2H), 6.43 (s, 1H), 5.57 (d, 2H), 5.30 (d, 2H), 3.01 (sp, 1H), 2.33 (s, 3H), 1.39 (d, 6H).

### Example 4

### [(η⁶-p-cymene)RuCl((CH₃)₃CCOCHCOC(CH₃)₃ - O,O)]

2,2,6,6-tetramethyl-3,5-heptanedione (299 mg, 1.62 mmol) and sodium methoxide (68 mg, 1.26 mmol) were stirred in methanol (40 ml) at RT for 5 h. The solvent and the excess liquid starting material were removed *in vacuo* on a rotary evaporator. The obtained sodium salt (233 mg, 1.13 mmol, 89.6% yield) and [(η⁶-*p*-cymene)RuCl₂]₂ (258 mg, 0.42 mmol) were stirred in acetone (25 ml) at RT for 40 min. After the extraction step using dichloromethane (see Example 1), the residue was dissolved in diethyl ether, concentrated on a rotary evaporator, and hexane was added. The solution was left in a freezer at 253 K overnight. The crystals (215 mg, 0.47 mmol, 56.0 % yield) were collected and dried *in vacuo*. ¹H NMR (CDCl₃): δ 5.38 (d, 2H), 5.37 (s, 1H), 5.10 (d, 2H), 2.88 (sp, 1H), 2.21 (s, 3H), 1.33 (d, 6H), 1.10 (s, 18H).

### Example 5

### [(η⁶-C₆H₅C₆H₅)RuCl(H₃CCOCHCOCH₃ - O,O)]

[(η⁶-C₆H₅C₆H₅)RuCl₂]₂ (250 mg, 0.38 mmol) was refluxed in water (25 ml) for 2 h. The heat was reduced below boiling point and sodium acetylacetonate monohydrate (141 mg, 1.01 mmol) was added. The solution was stirred for 30 min and hot filtered. The solvent was removed *in vacuo* on a rotary evaporator. After the extraction step using dichloromethane (see Example 1), the residue was dissolved in little acetone, and diethyl ether was added until precipitation occurred. The vessel was left in a freezer at 253 K for 6 d. The product (65 mg, 0.16 mmol, 21.3% yield) was collected and dried *in vacuo*. ¹H NMR (CDCl₃): δ 7.67 (m, 2H), 7.45 (m, 3H), 5.83 (m, 2H), 5.75 (m, 3H), 5.10 (s, 1H), 1.83 (s, 6H).

### Example 6

### [(η⁶-p-cymene)Ru(CH₃CD)(H₃CCOCHCOCH₃ - O,O)]BF₄

The compound of Example 1 (144 mg, 0.39 mmol) and AgBF₄ (72 mg, 0.37 mmol) were stirred in acetonitrile (20 ml) overnight. The resulting solution was filtered through glass wool and the solvent was removed *in vacuo* on a rotary evaporator. The residue was dissolved in a minimum amount of acetone, and diethyl ether was added until precipitation occurred. The flask was placed in a freezer at 253 K for 4 d. The crystals (149 mg, 0.32 mmol, 87.1% yield) were collected and dried *in vacuo*. ¹H NMR (CDCl₃): δ 5.74 (d, 2H), 5.48 (d, 2H), 5.16 (s, 1H), 2.85 (sp, 1H), 2.43 (s, 3H), 2.21 (s, 3H), 1.98 (s, 6H), 1.33 (d, 6H).

### Example 7

### [(η⁶-p-cymene)RuCl(H₃CCOCCOCH₃ - O,O)]₂

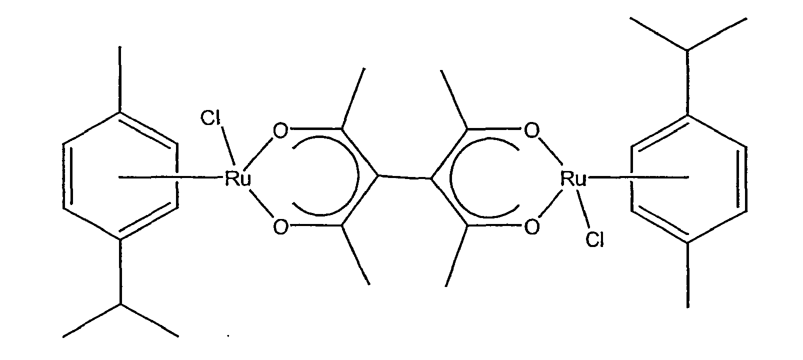

The preparation of the tetradentate ligand tetraacetylethane has been reported by Robert. G. Charles in Organic Syntheses (1959), 39, 61. Tetraacetylethane (208 mg, 1.05 mmol) was reacted in water with 21 ml of a 0.1 M solution of KOH in methanol for 2 h. The solvents were evaporated and the salt was dissolved in acetone. [(*p*-Cymene)RuCl₂]₂ (643 mg, 1.05 mmol) was added to the solution and the mixture was reacted for 3 h. The solvent was evaporated and the residue was extracted with CH₂Cl₂. Filtration and evaporation of the solvent gave the pure dinuclear complex, which was recrystallized from acetone (550 mg, 71%).

### B. Biological Data

### 1. Protocol for testing Ru compounds

The compounds are tested on 24-well trays. Cells growing in a flask are harvested just before they become confluent, counted using a haemocytometer and diluted down with media to a concentration of 1x10⁴ cells per ml. The cells are then seeded in the 24-well trays at a density of 1x10⁴ cells per well (i.e. 1ml of the diluted cell suspension is added to each well). The cells are then left to plate down and grow for 72 hours before adding the compounds of the invention.

The Ru complexes are weighed out and made up to a concentration of 1mg/ml with deionised water and 0.5 % w/w DMSO. The appropriate volume of the Ru solution is added to 5ml of media to make it up to a concentration of 100 µM for each drug. This 100 µM solution is then serially diluted to make up the 10 µM, 1 µM and 0.1 µM solutions.

The media is removed from the cells and replaced with 1ml of the media dosed with drug. Each concentration is done in duplicate. A set of control wells are left on each plate, containing media and 0.5 % w/w DMSO without drug.

The cells are left exposed to the drugs for 24 hours and then washed with phosphate buffered saline before fresh media is added.

They are allowed to grow on for a further 3 days before being counted using a Coulter counter.

### Preparing cells for counting:

Media is removed and 1ml of PBS is added to the cells.
250 µl of trypsin is added and cells left in incubator for a few minutes to allow the monolayers to detach.
Once trypsinised, 250µl of media is added to each well to neutralise the trypsin. 200µl of this suspension is added to 10ml of NaCl for counting.

### 2. Results

Using the above protocol, a number of compounds of the invention were tested on A2780 ovarian cancer cell line. Compounds of the invention have an IC50 of less than 150 µM, preferably less than 100 µM, more preferably less than 50 µM when tested according to this protocol. The results are as follows:

| Compound (Example No.) | IC50 (µM) |
|---|---|
| 1 | 19 |
| 2 | >100 |
| 3 | 11 |
| 4 | 14 |
| 5 | 21 |
| 6 | 22 |
| 7 | - |

### C. NMR Data

The ability of the compounds of the invention to bind to DNA bases was studied by nmr and the results are illustrated in the accompanying figures in which:

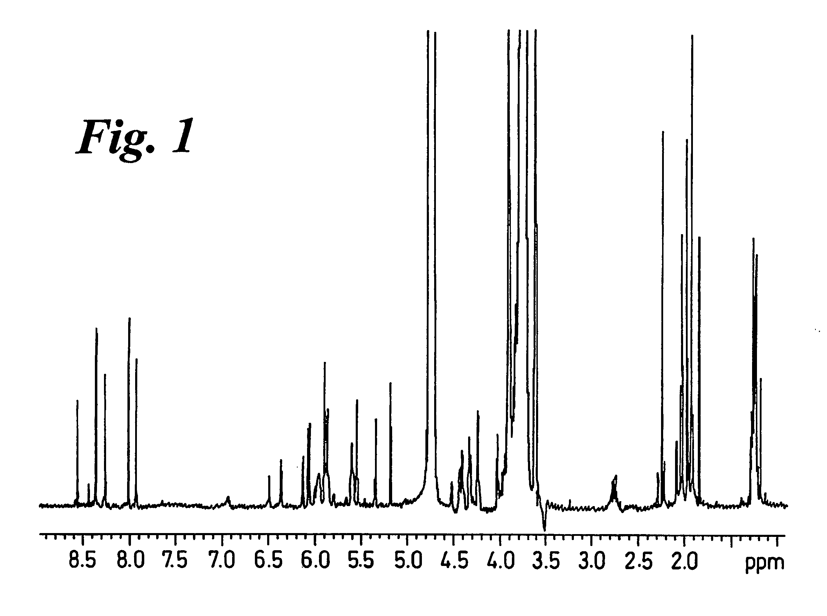

Figure 1 is a 500 MHz ¹H NMR spectrum of a mixture of guanosine : adenosine : compound of Example 1 in the mole ratio 1.3 : 1 : 1, respectively, in 10% D₂O/90% H₂O.
Figure 2 shows expansions of the regions 5.1-5.4 ppm and 7.9-8.6 ppm of the ¹H NMR spectrum shown in Fig.1.

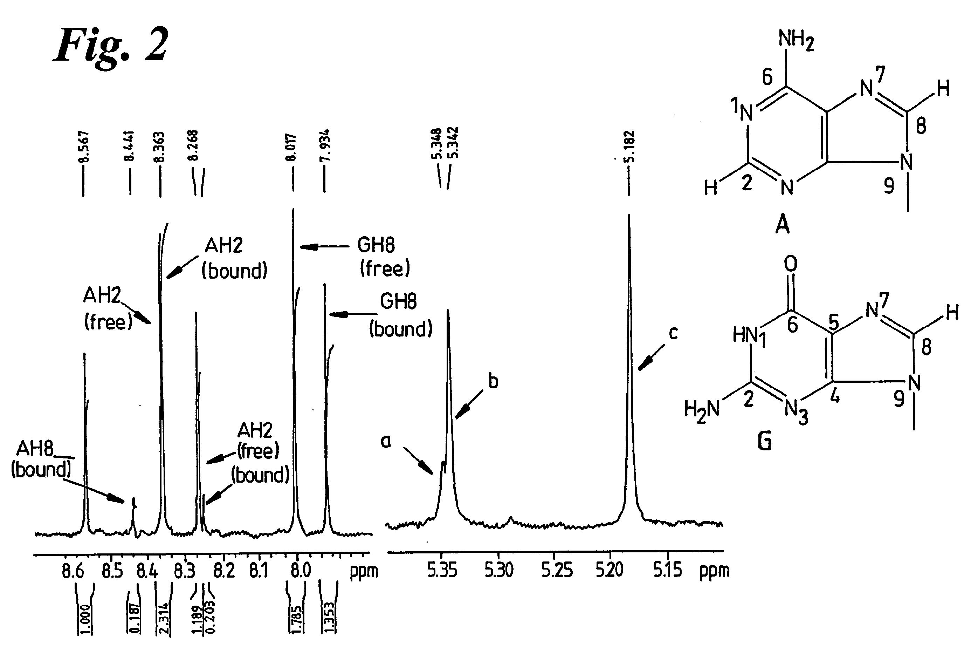

In Figure 2, the region from 5.1 to 5.4 ppm (right) corresponds to the central CH on the acac ligand. Peaks a and b correspond to the adenosine adducts, peak c to the guanosine adduct. In the region 7.9- 8.6 ppm (left), peaks for the H2 and H8 protons of adenosine and H8 protons of guanosine appear. It can be seen that both adenosine and guanosine form adducts with the compound of Example 1 (bound labels). The atom labelling scheme for the nucleobase part (A and G) of the nucleosides is shown.

## Claims

1. Ruthenium(II) compound of formula(I) : 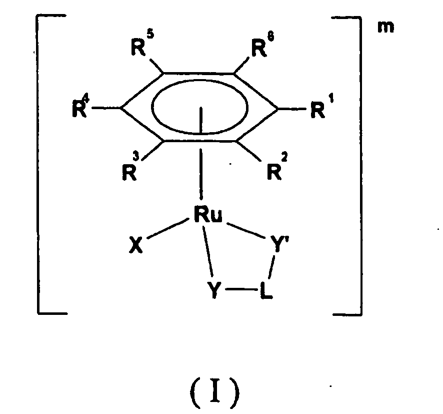 wherein: R¹, R², R³, R⁴, R⁵ and R⁶ independently represent H, (C₁-C₆)alkyl, (C₂-C₆)alkenyl, (C₂-C₆)alkynyl, hydroxy(C₁-C₆)alkyl, amino(C₁-C₆)alkyl, halo, CO₂R⁷, CONR⁸R⁹, COR¹⁰, SO₃H, SO₂NR¹¹R¹², aryloxy, (C₁-C₆)alkoxy, (C₁-C₆)alkylthio, -N=N-R¹³, NR¹⁴R¹⁵, aryl or aralkyl, which latter two groups are optionally substituted on the aromatic ring by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7a}, CONR^{8a}R^{9a}, COR^{10a}, SO₃G, SO₂NR^{11a}R^{12a}, aryloxy, (C₁-C₆)alkoxy, (C₁-C₆)alkylthio, -N=N-R^{13a}, NR^{14a}R^{15a}, or R¹ and R² together with the ring to which they are bound represent a saturated or unsaturated carbocyclic or heterocyclic group containing up to three 3-to 8-membered carbocyclic or heterocyclic rings, wherein each carbocyclic or heterocyclic ring may be fused to one or more other carbocyclic or heterocyclic rings, and wherein each of the rings may be optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R^{15b} or (C₁C₆)alkoxy;
R⁷, R⁸, R⁹, R¹⁰, R¹¹, R¹², R¹³, R¹⁴, R¹⁵, R^{7a}, R^{8a}, R^{9a}, R^{10a}, R^{11a}, R^{12a}, R^{13a}, R^{14a}, R^{15a}, R^{7b}, R^{8b}, R^{9b}, R^{10b}, R^{11b}, R^{12b}, R^{13b}, R^{14b}, and R^{15b} are independently selected from H, (C₁-C₆)alkyl, aryl or aralkyl;
X is a neutral or negatively charged O-, N- or S-donor ligand or halo;
G and G' are independently selected from alkali metals, aryl, aralkyl and (C₁-C₆) alkyl;
Y-L-Y' is a bidentate ligand bearing a negative charge with a proportion of the charge on both Y and Y', Y and Y' are independently selected from O, S or NR¹⁶, wherein R¹⁶ is H, (C₁-C₆) alkyl, aryl or aralkyl, and L is a group linking Y and Y' and comprises one or more groups selected from (C₁-C₆) alkylene, (C₁-C₆) alkenylene, (C₁-C₆) alkynylene, arylene, aralkylene, alkarylene, each of said latter six groups being optionally substituted, ferrocenylene, Se, Se-Se, S-S, N=N and C=O;
m is -1, 0 or +1 and the compound comprises a counterion when m is -1 or +1; the compound of formula (I) optionally being in the form of a dimer in which two L groups are linked either directly or through a group comprising one or more of (C₁-C₆) alkylene, (C₁-C₆) alkenylene, arylene, aralkylene, alkarylene, Se, Se-Se, S-S, N=N and C=O or in which L bears two Y groups and two Y' groups;
with the provisos that:
when Y-L-Y' is (CH₃C(O)CHC(O)CH₃)⁻, X is halo or an N-donor ligand, R¹, R², R³, R⁴, R⁵ and R⁶ together with the ring to which they are bound do not
represent 4-isopropyl-1-methylbenzene;
when Y-L-Y' is (CH₃C(O)CHC(O)CH₃)⁻ and X is chloro, (CH₃)₂SO, CH₃CN, pyridine or (CH₃C(O)CHC(O)CH₃)⁻: R¹, R², R³, R⁴, and R⁶ are not all H or all methyl; R¹, R³ and R⁵ are not all H when R², R⁴ and R⁶ are all methyl; and R², R⁴ and R⁶ are not all H when R¹, R³ and R⁵ are all methyl;
when Y-L-Y' is (CF₃C(O)CHC(O)CHF₃)⁻ and X is chloro, R¹, R², R³, R⁴, R⁵ and R⁶ are not all H or all methyl; R¹, R³ and R⁵ are not all H when R², R⁴ and R⁶ are all methyl; and R², R⁴ and R⁶ are not all H when R¹, R³ and R⁵ are all methyl;
when Y-L-Y' is (CF₃C(O)CHC(O)OEt)⁻ and X is chloro, R¹, R², R³, R⁴, R⁵ and R⁶ together with the ring to which they are bound do not represent 4-isopropyl-1-methylbenzene;
when Y-L-Y' is ((ferrocenylene)C(O)CHC(O)(ferrocenylene))⁻ and X is chloro, R¹, R², R³, R⁴, R⁵ and R⁶ together with the ring to which they are bound do not represent 4-isopropyl-1-methylbenzene;
when R¹, R², R³, R⁴, R⁵ and R⁶ together with the ring to which they are bound represent 4-isopropyl-1-methylbenzene and X is chloro, Y-L-Y' is neither (3-OCH₃-4-OH-PhCHCHC(O)CHC(O)CHCH-3-OCH₃-4-OH-Ph)⁻, (3-OCH₃-4-OCH₃-PhCHCHC(O)C(CH₃)C(O)CHCH-3-OCH₃-4-OCH₃-Ph)⁻, (3-OCH₃-4-COOCH₃-PhCHCHC(O)CHC(O)CHCH-3-OCH₃-4-COOCH₃-Ph)⁻, (4-OH-PhCHCHC(O)CHC(O)CHCH-4-OH-Ph)⁻ nor (3-OCH₃-4-OCH₃-PhCHCHC(O)CHC(O)CHCH-3-OCH₃-4-OCH₃-Ph)⁻.

2. Compound as claimed in Claim 1, wherein R¹, R², R³, R⁴, R⁵ and R⁶ are independently selected from H, (C₁-C₆) alkyl and phenyl or R¹ and R² together with the ring to which they are bound represent anthracene or a hydrogenated derivative of anthracene, said phenyl and anthracene or a hydrogenated derivative of anthracene group being optionally substituted by one or more groups independently selected from (C₁-C₆)alkyl, (C₂-C₆) alkenyl, (C₂-C₆)alkynyl, hydroxy(C₁-C₆)alkyl, amino(C₁-C₆)alkyl, phenyl, benzyl, halo, carboxyl, CO₂(C₁-C₆)alkyl, CONH₂, COH, CO(C₁-C₆)alkyl, SO₃H, SO₂NH₂, phenoxy, (C₁-C₆)alkylthio, NH₂ or (C₁-C₆) alkoxy.

3. Compound as claimed in Claim 1 or Claim 2, wherein m is 0.

4. Compound as claimed in any one of Claims 1 to 3, wherein X is halo or CH₃CN.

5. Compound as claimed in any one of Claims 1 to 4, wherein Y-L-Y' is selected from ligands of formulae (II) to (X): 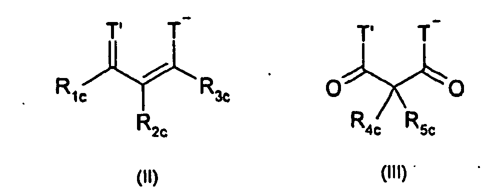 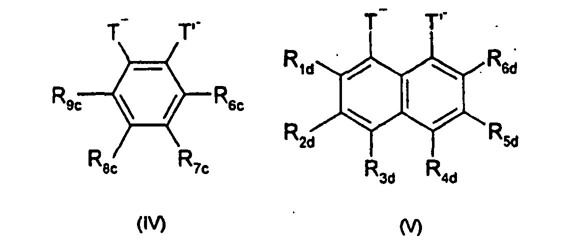 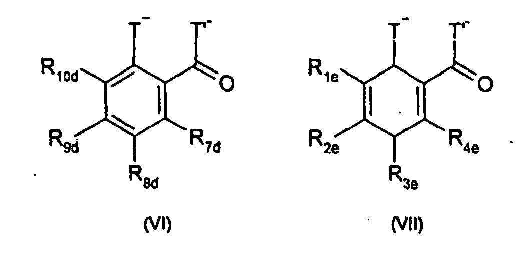 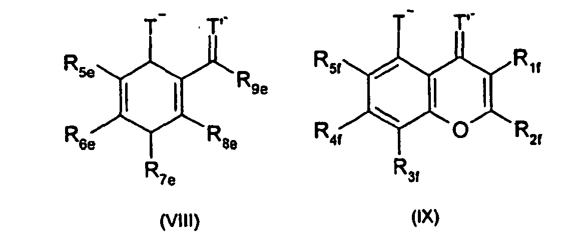 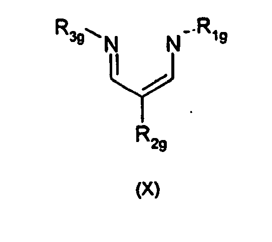 wherein T and T' are independently selected from O and S,
R_{1g} and R_{3g} are independently H, (C₁-C₆) alkyl, aryl or aralkyl,
R_{1c} to R_{5f} and R_{2g} are independently H, (C₁-C₆)alkyl, aryl, aralkyl, wherein the latter two groups and the corresponding groups for R_{1g} and R_{3g} are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R^{15b} or (C₁- C₆)alkoxy, wherein R^{7b}, R^{8b}, R^{9b}, R^{10b}, R^{11b}, R^{12b}, R^{13b}, R^{14b}, and R^{15b} are as defined in Claim 1.

6. Compound as claimed in any one of Claims 1 to 4, wherein Y-L-Y' is selected from: 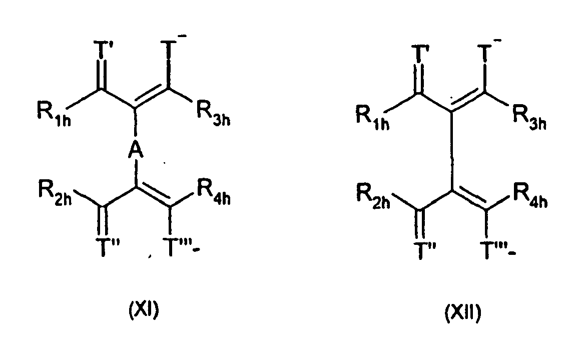 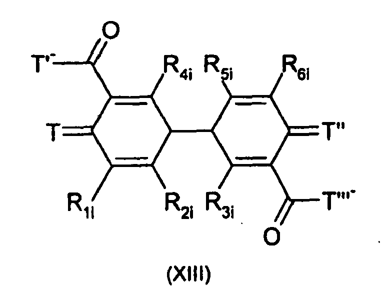 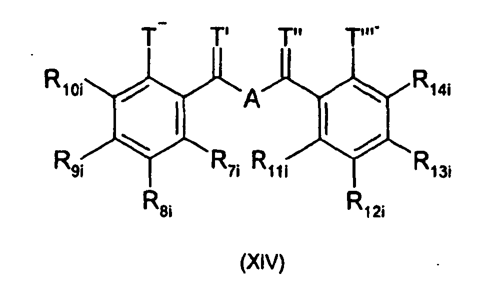 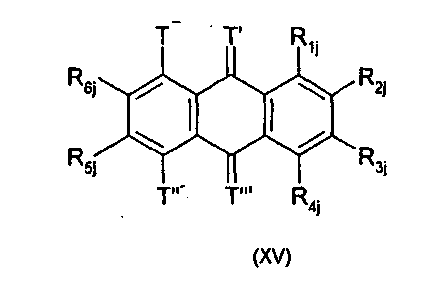 wherein T, T', T" and T''' are independently selected from O and S,
A comprises one or more groups selected from (C₁-C₆)alkylene, (C₁-C₆)alkenylene, (C₁-C₆) alkynylene, arylene, aralkylene, alkarylene, ferrocenylene, Se, Se-Se, S-S, N=N and C=O
and R₁ₕ to R₆ⱼ are independently H, (C₁-C₆)alkyl, aryl, aralkyl, wherein the latter two groups are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R^{15b} or (C₁- C6)alkoxy, wherein R^{7b}, R^{8b}, R^{9b}, R^{10b}, R^{11b}, R^{12b}, R^{13b}, R^{14b}, and R^{15b} are as defined in Claim 1.

7. Compound as claimed in any one of Claims 1 to 4, wherein Y-L-Y' is: 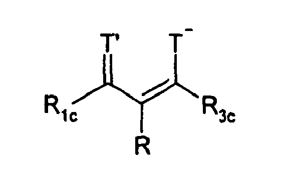 wherein T and T' are independently O and S, and R, R_{1c}, and R_{3c} are independently H, (C₁-C₆)alkyl, aryl, aralkyl, wherein the latter two groups are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R¹⁵b or (C₁-C₆)alkoxy, wherein R^{7b}, R^{8b}, R^{9b}, R^{10b}, R^{11b}, R^{12b}, R^{13b}, R^{14b}, and R^{15b} are as defined in Claim 1.

8. Compound as claimed in claim 7, wherein one of R¹, R², R³, R⁴, R⁵ and R⁶ is phenyl and the other groups are H.

9. Compound as claimed in Claim 7 or Claim 8, wherein T and T' are both O, R is H or (C₁-C₆) alkyl and R_{1c} and R_{3c} are independently (C₁-C₆)alkyl or phenyl, said phenyl optionally substituted by (C₁-C₆)alkyl, hydroxy(C₁-C₆)alkyl, amino(C₁-C₆) alkyl, halo, carboxyl, CO₂(C₁-C₆)alkyl, CONH₂, COH, CO(C₁-C₆)alkyl, SO₃H, SO₂NH₂, phenoxy, (C₁-C₆) alkylthio, NH₂ or (C₁-C₆)alkoxy.

10. Compound as claimed in claim 9, wherein R_{1c} and R_{3c} are independently phenyl, said phenyl optionally substituted by (C₁-C₆)alkyl, hydroxy(C₁-C₆)alkyl, amino(C₁-C₆) alkyl, halo, carboxyl, CO₂(C₁-C₆)alkyl, CONH₂, COH, CO(C₁-C₆)alkyl, SO₃H, SO₂NH₂, phenoxy, (C₁-C₆) alkylthio, NH₂ or (C₁-C₆)alkoxy.

11. Compound as claimed in claim 9, wherein R is H and R_{1c} and R_{3c} are independently (C₁-C₆)alkyl or phenyl.

12. Compound as claimed in any one of Claims 1 to 9, wherein Y and Y' are both O.

13. Ruthenium(II) compound of formula(I) : 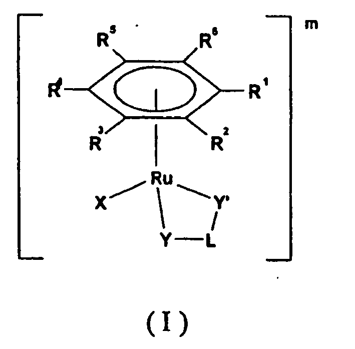 for use in therapy wherein:
R¹, R², R³, R⁴, R⁵ and R⁶ independently represent H, (C₁-C₆)alkyl, (C₂-C₆)alkenyl, (C₂-C₆)alkynyl, hydroxy(C₁-C₆)alkyl, amino(C₁-C₆)alkyl, halo, CO₂R⁷, CONR⁸R⁹, COR¹⁰, SO₃H, SO₂NR¹¹R¹², aryloxy, (C₁-C₆)alkoxy, (C₁-C₆)alkylthio, -N=N-R¹³, NR¹⁴R¹⁵, aryl or aralkyl, which latter two groups are optionally substituted on the aromatic ring by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7a}, CONR^{8a}R^{9a}, COR^{10a}, SO₃G, SO₂NR^{11a}R^{12a}, aryloxy, (C₁-C6)alkoxy, (C₁-C₆)alkylthio, -N=N-R^{13a}, NR^{14a}R^{15a}, or R¹ and R² together with the ring to which they are bound represent a saturated or unsaturated carbocyclic or heterocyclic group containing up to three 3-to 8-membered carbocyclic or heterocyclic rings, wherein each carbocyclic or heterocyclic ring may be fused to one or more other carbocyclic or heterocyclic rings, and wherein each of the rings may be optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R^{15b} or (C₁-C₆)alkoxy;
R⁷, R⁸, R⁹, R¹⁰, R¹¹, R¹², R¹³, R¹⁴, R¹⁵, R^{7a}, R^{8a}, R^{9a}, R^{10a}, R^{11a}, R^{11a}, R^{13a}, R^{14a}, R^{15a}, R^{7b}, R^{8b}, R^{9b}, R^{10b}, R^{11b}, R^{12b}, R^{13b}, R^{14b}, and R^{15b} are independently selected from H, (C₁-C₆)alkyl, aryl or aralkyl;
X is a neutral or negatively charged O-, N- or S-donor ligand or halo;
G and G' are independently selected from alkali metals, aryl, aralkyl and (C₁-C₆) alkyl;
Y-L-Y' is a bidentate ligand bearing a negative charge with a proportion of the charge on both Y and Y', Y and Y' are independently selected from O, S or NR¹⁶, wherein R¹⁶ is H, (C₁-C₆) alkyl, aryl or aralkyl, and L is a group linking Y and Y' and comprises one or more groups selected from (C₁-C₆) alkylene, (C₁-C₆) alkenylene, (C₁-C₆) alkynylene, arylene, aralkylene, alkarylene, each of said latter six groups being optionally substituted, ferrocenylene, Se, Se-Se, S-S, N=N and C=O;
m is -1, 0 or +1 and the compound comprises a counterion when m is -1 or +1; the compound of formula (I) optionally being in the form of a dimer in which two L groups are linked either directly or through a group comprising one or more of (C₁-C₆) alkylene, (C₁-C₆) alkenylene, arylene, aralkylene, alkarylene, Se, Se-Se, S-S, N=N and C=O or in which L bears two Y groups and two Y' groups.

14. Compound as claimed in Claim 13, wherein R¹, R², R³, R⁴, R⁵ and R⁶ are independently selected from H, (C₁-C₆) alkyl and phenyl or R¹ and R² together with the ring to which they are bound represent anthracene or a hydrogenated derivative of anthracene, said phenyl and anthracene or a hydrogenated derivative of anthracene group being optionally substituted by one or more groups independently selected from (C₁-C₆)alkyl, (C₂-C₆) alkenyl, (C₂-C₆)alkynyl, hydroxy(C₁-C₆)alkyl, amino(C₁-C₆)alkyl, phenyl, benzyl, halo, carboxyl, CO₂(C₁-C₆)alkyl, CONH₂, COH, CO(C₁-C₆)alkyl, SO₃H, SO₂NH₂, phenoxy, (C₁-C₆)alkylthio, NH₂ or (C₁-C₆) alkoxy.

15. Compound as claimed in Claim 13 or Claim 14, wherein m is 0.

16. Compound as claimed in any one of Claims 13 to 15, wherein X is halo or CH₃CN.

17. Compound as claimed in any one of Claims 13 to 16, wherein Y-L-Y' is selected from ligands of formulae (II) to (X): 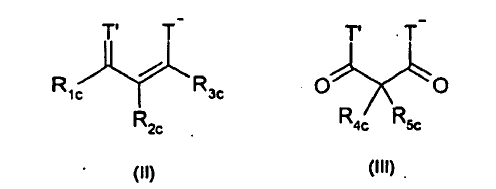 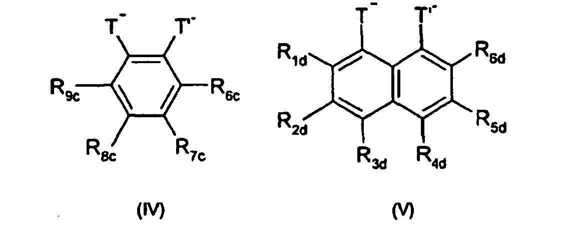 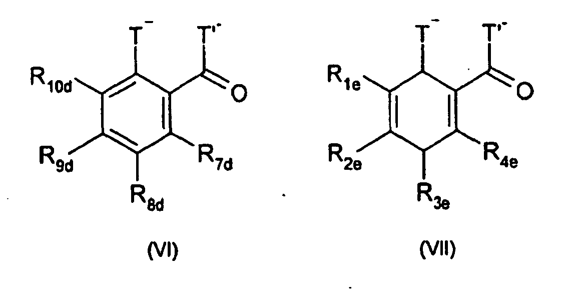 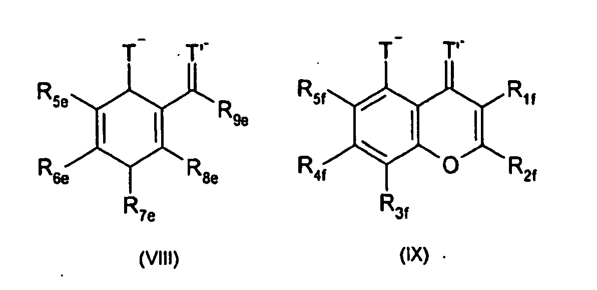 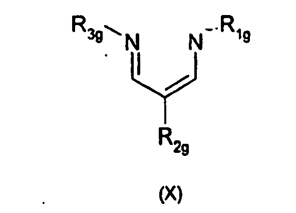 wherein T and T' are independently selected from O and S,
R_{1g} and R_{3g} are independently H, (C₁-C₆) alkyl, aryl or aralkyl,
R_{1c} to R_{5f} and R_{2g} are independently H, (C₁-C₆)alkyl, aryl, aralkyl, wherein the latter two groups and the corresponding groups for R_{1g} and R_{3g} are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R^{15b} or (C₁- C₆)alkoₓy, wherein R^{7b}, R^{8b}, R^{9b}, R^{10b}, R^{11b}, R^{12b}, R^{13b}, R^{14b}, and R^{15b} are as defined in Claim 13.

18. Compound as claimed in any one of Claims 13 to 16, wherein Y-L-Y' is selected from: 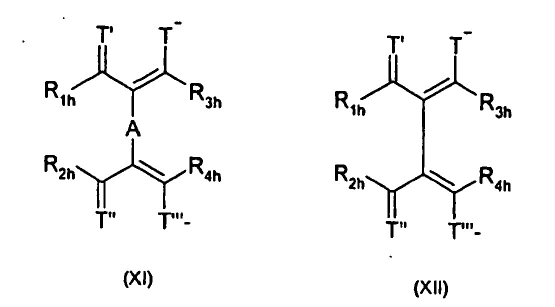 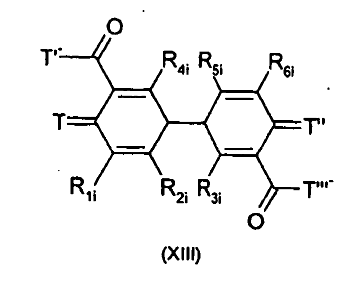 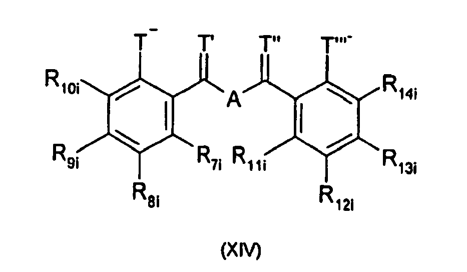 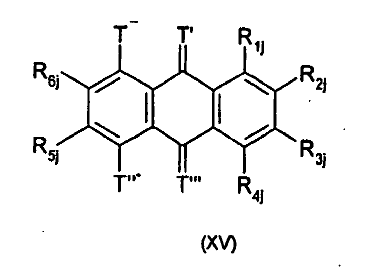 wherein T, T', T" and T''' are independently selected from O and S,
A comprises one or more groups selected from (C₁-C₆)alkylene, (C₁-C₆)alkenylene, (C₁-C₆) alkynylene, arylene, aralkylene, alkarylene, ferrocenylene, Se, Se-Se, S-S, N=N and C=O
and R₁ₕ to R₆ⱼ are independently H, (C₁-C₆)alkyl, aryl, aralkyl, wherein the latter two groups are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{11b}, SO₃G', SO₂NR^{11b}R^{12b}, aryloxy, (C₁-C₆)alkylthio, -N=N-R^{13b}, NR^{14b}R^{15b} or (C₁- C₆)alkoxy, wherein R^{7b}, R^{8b}, R^{9b}, R^{10b}, R^{11b}, R^{12b}, R^{13b}, R^{14b}, and R^{15b} are as defined in Claim 13.

19. Compound as claimed in any one of Claims 13 to 16, wherein Y-L-Y' is: 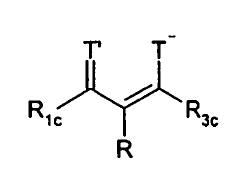 wherein T and T' are independently O and S, and R, R_{1c}, and R_{3c} are independently H, (C₁-C₆)alkyl, aryl, aralkyl, wherein the latter two groups are optionally substituted by one or more groups independently selected from (C₁-C₆)alkyl, (C₂-C₆)alkenyl, (C₂-C₆)alkynyl, hydroxy(C₁-C₆)alkyl, amino(C₁-C₆)alkyl, aryl, aralkyl, halo, CO₂R^{7b}, CONR^{8b}R^{9b}, COR^{10b}, SO₃G', SO₂NR^{11b}R^{12b}, aryloxy, (C₁-C₆)alkylthio, -N=N-R^{13b}, NR^{14b}R^{11b} or (C₁-C₆)alkoxy, wherein R^{7b}, R^{8b}, R^{9b}, R^{10b}, R^{11b}, R^{12b}, R^{13b}, R^{14b}, and R^{15b} are as defined in Claim 13.

20. Compound as claimed in Claim 19, wherein T and T' are both 0, R is H or (C₁-C₆) alkyl and R_{1c} and R_{3c} are independently (C₁-C₆)alkyl or phenyl, said phenyl optionally substituted by (C₁-C₆)alkyl, hydroxy(C₁-C₆)alkyl, amino(C₁-C₆) alkyl, halo, carboxyl, CO₂(C₁-C₆)alkyl, CONH₂, COH, CO(C₁-C₆)alkyl, SO₃H, SO₂NH₂, phenoxy, (C₁-C₆) alkylthio, NH₂ or (C₁-C₆)alkoxy.

21. Compound as claimed in claim 20, wherein R is H and R_{1c} and R_{3c} are independently (C₁-C₆)alkyl or phenyl.

22. Compound as claimed in any one of Claims 13 to 21, wherein Y and Y' are both O.

23. Use of a compound of formula (I) according to any one of Claims 13 to 22, in the manufacture of a medicament for the treatment and/or prevention of cancer.

24. Pharmaceutical composition comprising a compound of formula (I) according to any one of Claims 13 to 21, together with one or more pharmaceutically acceptable excipients.

25. A compound of formula (I) according to any one of Claims 13 to 21 or a composition of Claim 24, for use in the that ment and/or prevention of cancer.

26. Process for preparing the compound of any one of Claims 1 to 12 which comprises the reaction of a compound of formula [(η⁶-C₆(R¹)(R²)(R³)(R⁴)(R⁵)(R⁶)) RuX₂], optionally in the form of a dimer, with Y-L-Y, in a suitable solvent for the reaction, wherein R¹, R², R³, R⁴, R⁵, R⁶, X, Y,Y' and L are as defined in Claim 1.

## Patentansprüche

1. Ruthenium(II)-Verbindung der Formel (I): 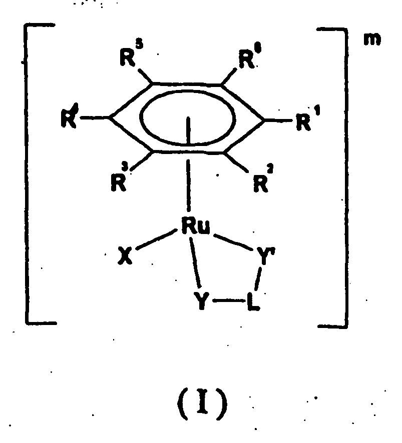 worin:
R¹, R², R³, R⁴, R⁵ und R⁶ unabhängig voneinander für H, (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Halogen, CO₂R⁷, CONR⁸R⁹, COR¹⁰, SO₃H, SO₂NR¹¹R¹², Aryloxy, (C₁-C₆)-Alkoxy, (C₁-C₆)-Alkylthio, -N=N-R¹³, NR¹⁴R¹⁵, Aryl oder Aralkyl stehen, wobei die letzteren beiden Gruppen gegebenenfalls am aromatischen Ring mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7a}, CONR^{8a}R^{9a}, COR^{10a}, SO₃G, SO₂NR^{11a}R^{12a}, Aryloxy, (C₁-C₆)-Alkoxy, (C₁-C₆)-Alkylthio, -N=N-R^{13a}, NR^{14a}R^{15a} ausgewählten Gruppen substituiert sind, oder R¹ und R² gemeinsam mit dem Ring, an den sie gebunden sind, eine gesättigte oder ungesättigte carbozyklische oder heterozyklische Gruppe mit bis zu drei 3- bis 8-gliedrigen carbozyklischen oder heterozyklischen Ringen darstellen, worin jeder carbozyklische oder heterozyklische Ring gegebenenfalls an einen oder mehrere andere carbozyklische oder heterozyklische Ringe kondensiert ist und worin jeder der Ringe gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b}, CONR^{8b}R^{9b}, COR^{10b}, SO₃G', SO₂NR^{11b}R^{12b}, Aryloxy, (C₁-C₆)-Alkylthio, -N=N-R^{13b}, NR^{14b}R^{15b} oder (C₁-C₆)-Alkoxy ausgewählten Gruppen substituiert ist;
R⁷, R⁸, R⁹, R¹⁰, R¹¹, R¹², R¹³, R¹⁴, R¹⁵, R^{7a}, R^{8a}, R^{9a}, R^{10a}, R^{11a}, R^{12a}, R^{13a}, R^{14a}, R^{15a}, R^{7b}, R^{8b}, R^{9b}, R^{10b}, R^{11b}, R^{12b}, R^{13b}, R^{14b} und R^{15b} unabhängig voneinander aus H, (C₁-C₆)-Alkyl, Aryl oder Aralkyl ausgewählt sind;
X ein neutraler oder negativ geladener O-, N- oder S-Donorligand oder ein Halogen ist;
G und G' unabhängig voneinander aus Alkalimetallen, Aryl, Aralkyl und (C₁-C₆)-Alkyl ausgewählt sind;
Y-L-Y' ein zweizähniger Ligand ist, der eine negative Ladung trägt, wobei ein Teil der Ladung auf sowohl Y als auch Y' vorliegt, Y und Y' unabhängig voneinander aus O, S oder NR¹⁶ ausgewählt sind, worin R¹⁶ H, (C₁-C₆)-Alkyl, Aryl oder Aralkyl ist, und L eine Gruppe ist, die Y und Y' verbindet und eine oder mehrere aus (C₁-C₆)-Alkylen, (C₁-C₆)-Alkenylen, (C₁-C₆)-Alkinylen, Arylen, Aralkylen, Alkarylen, wobei jede der letzteren sechs Gruppen gegebenenfalls substituiert ist, Ferrocenylen, Se, Se-Se, S-S, N=N und C=O ausgewählte Gruppen umfasst;
m = -1, 0 oder +1 ist und die Verbindung ein Gegenion umfasst, wenn m -1 oder +1 ist;
wobei die Verbindung der Formel (I) gegebenenfalls in Form eines Dimers vorliegt, worin zwei L-Gruppen entweder direkt oder durch eine Gruppe verbunden sind, die eines oder mehrere aus (C₁-C₆)-Alkylen, (C₁-C₆)-Alkenylen, Arylen, Aralkylen, Alkarylen, Se, Se-Se, S-S, N=N und C=O umfasst, oder worin L zwei Y-Gruppen und zwei Y'-Gruppen trägt;
mit der Maßgabe, dass
wenn Y-L-Y' (CH₃C(O)CHC(O)CH₃)⁻ ist, X Halogen oder ein N-Donorligand ist, R¹, R², R³, R⁴, R⁵ und R⁶ gemeinsam mit dem Ring, an den sie gebunden sind, nicht für 4-Isopropyl-1-methylbenzol stehen;
wenn Y-L-Y' (CH₃C(O)CHC(O)CH₃)⁻ ist und X Chlor, (CH₃)₂SO, CH₃CN, Pyridin oder (CH₃C(O)CHC(O)CH₃)⁻ ist, R¹, R², R³, R⁴, R⁵ und R⁶ nicht allesamt H oder Methyl sind; R¹, R³ und R⁵ nicht allesamt H sind, wenn R², R⁴ und R⁶ allesamt Methyl sind; und R², R⁴ und R⁶ nicht allesamt H sind, wenn R¹, R³ und R⁵ allesamt Methyl sind;
wenn Y-L-Y' (CF₃C(O)CHC(O)CHF₃)⁻ ist und X Chlor ist, R¹, R², R³, R⁴, R⁵ und R⁶ nicht allesamt H oder Methyl sind; R¹, R³ und R⁵ nicht allesamt H sind, wenn R², R⁴ und R⁶ allesamt Methyl sind; und R², R⁴ und R⁶ nicht allesamt H sind, wenn R¹, R³ und R⁵ allesamt Methyl sind;
wenn Y-L-Y' (CF₃C(O)CHC(O)OEt)⁻ ist und X Chlor ist, R¹, R², R³, R⁴, R⁵ und R⁶ gemeinsam mit dem Ring, an den sie gebunden sind, nicht für 4-Isopropyl-1-methylbenzol stehen;
wenn Y-L-Y' ((Ferrocenylen)C(O)CHC(O)(ferrocenylen))⁻ ist und X Chlor ist, R¹, R², R³, R⁴, R⁵ und R⁶ gemeinsam mit dem Ring, an den sie gebunden sind, nicht für 4-Isopropyl-1-methylbenzol stehen;
wenn R¹, R², R³, R⁴, R⁵ und R⁶ gemeinsam mit dem Ring, an den sie gebunden sind, für 4-Isopropyl-1-methylbenzol stehen und X Chlor ist, Y-L-Y' weder (3-OCH₃-4-OH-PhCHCHC(O)CHC(O)CHC-3-OCH₃-4-OH-Ph)⁻, (3-OCH₃-4-OCH₃-PhCHCHC(O)C(CH₃)C(O)CHCH-3-OCH₃-4-OCH₃-Ph)⁻, (3-OCH₃-4-COOCH₃-PhCHCHC(O)CHC(O)CHCH-3-OCH₃-4-COOCH₃-Ph)⁻, (4-OH-PhCHCHC(O)CHC(O)CHCH-4-OH-Ph)⁻ noch (3-OCH₃-4-OCH₃-PhCHCHC(O)CHC(O)CHCH-3-OCH₃-4-OCH₃-Ph)⁻ ist.

2. Verbindung nach Anspruch 1, worin R¹, R², R³, R⁴, R⁵ und R⁶ unabhängig voneinander aus H, (C₁-C₆)-Alkyl und Phenyl ausgewählt sind oder R¹ und R² gemeinsam mit dem Ring, an den sie gebunden sind, für Anthracen oder ein hydriertes Derivat von Anthracen stehen, wobei das Phenyl und Anthracen oder hydrierte Derivat der Anthracengruppe gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Phenyl, Benzyl, Halogen, Carboxyl, CO₂-(C₁-C₆)-alkyl, CONH₂, COH, CO-(C₁-C₆)-alkyl, SO₃H, SO₂NH₂, Phenoxy, (C₁-C₆)-Alkylthio, NH₂ oder (C₁-C₆)-Alkoxy ausgewählten Gruppen substituiert sind.

3. Verbindung nach Anspruch 1 oder Anspruch 2, worin m = 0 ist.

4. Verbindung nach einem der Ansprüche 1 bis 3, worin X ein Halogen oder CH₃CN ist.

5. Verbindung nach einem der Ansprüche 1 bis 4, worin Y-L-Y' aus Liganden der Formeln (II) bis (X) ausgewählt ist: 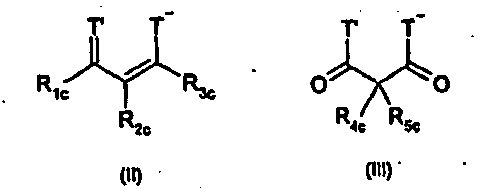 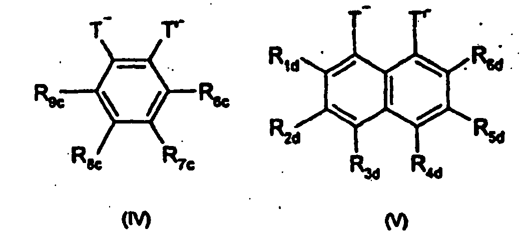 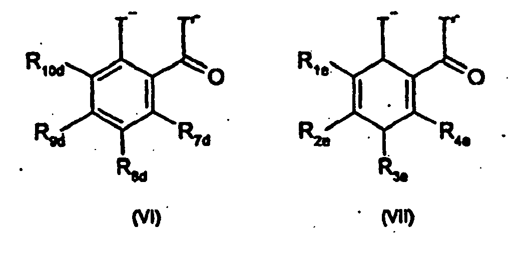 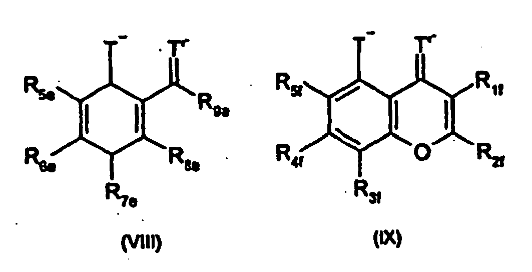 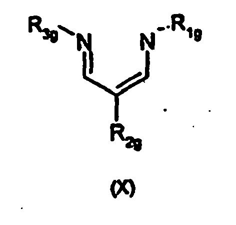 worin T und T' unabhängig voneinander aus O und S ausgewählt sind,
R_{1g} und R_{3g} unabhängig voneinander H, (C₁-C₆)-Alkyl, Aryl oder Aralkyl sind,
R_{1c} bis R_{5f} und R_{2g} unabhängig voneinander H, (C₁-C₆)-Alkyl, Aryl, Aralkyl sind, worin die letzteren beiden Gruppen und die entsprechenden Gruppen für R_{1g} und R_{3g} gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b}, CONR^{8b}R^{9b}, COR^{10b}, SO₃G', SO₂NR^{11b}R^{12b}, Aryloxy, (C₁-C₆)-Alkylthio, -N=N-R^{13b}, NR^{14b}R^{15b} oder (C₁-C₆)-Alkoxy, worin R^{7b}, R^{8b}, R^{9b}, R^{10b}, R^{11b}, R^{12b}, R^{13b}, R^{14b} und R^{15b} wie in Anspruch 1 definiert sind, ausgewählten Gruppen substituiert sind.

6. Verbindung nach einem der Ansprüche 1 bis 4, worin Y-L-Y' ausgewählt ist aus: 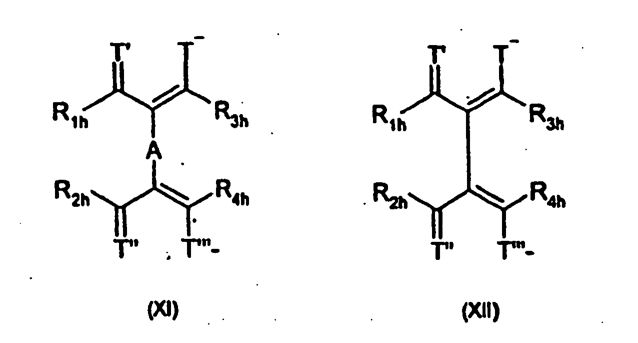 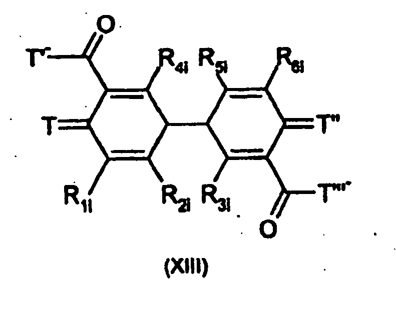 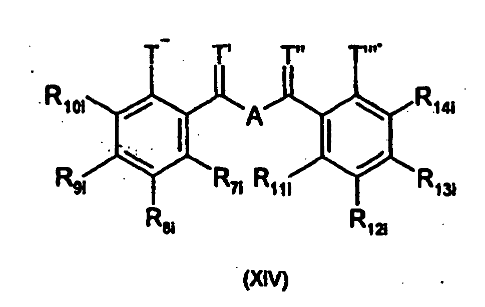 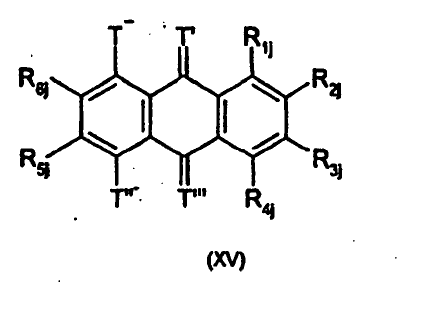 worin T, T', T" und T"' unabhängig voneinander aus O und S ausgewählt sind,
A eine oder mehrere aus (C₁-C₆)-Alkylen, (C₁-C₆)-Alkenylen, (C₁-C₆)-Alkinylen, Arylen, Aralkylen, Alkarylen, Ferrocenylen, Se, Se-Se, S-S, N=N und C=O ausgewählte Gruppen umfasst
und R₁ₕ bis R₆ⱼ unabhängig voneinander H, (C₁-C₆)-Alkyl, Aryl, Aralkyl sind, worin die letzteren beiden Gruppen gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b}, CONR^{8b}R^{9b}, COR^{10b}, SO₃G', SO₂NR^{11b}R^{12b}, Aryloxy, (C₁-C₆)-Alkylthio, -N=N-R^{13b}, NR^{14b}R^{15b} oder (C₁-C₆)-Alkoxy ausgewählten Gruppen substituiert sind, worin R^{7b}, R^{8b}, R^{9b}, R^{10b}, R^{11b}, R^{12b}, R^{13b}, R^{14b} und R^{15b} wie in Anspruch 1 definiert sind.

7. Verbindung nach einem der Ansprüche 1 bis 4, worin Y-L-Y' 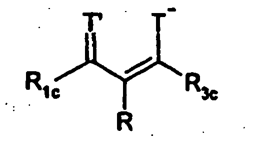 ist,
worin T und T' unabhängig voneinander O und S sind und R, R_{1c} und R_{3c} unabhängig voneinander H, (C₁-C₆)-Alkyl, Aryl, Aralkyl sind, worin die letzteren beiden Gruppen gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b} CONR^{8b}R^{9b} COR^{10b}, SO₃G', SO₂NR^{11b}R^{12b}, Aryloxy, (C₁-C₆)-Alkylthio, -N=N-R^{13b}, NR^{14b}R^{15b} oder (C₁-C₆)-Alkoxy ausgewählten Gruppen substituiert sind, worin R^{7b}, R^{8b}, R^{9b}, R^{10b}, R^{11b}, R^{12b}, R^{13b}, R^{14b} und R^{15b} wie in Anspruch 1 definiert sind.

8. Verbindung nach Anspruch 7, worin eines aus R¹, R², R³, R⁴, R⁵ und R⁶ Phenyl ist und die anderen Gruppen H sind.

9. Verbindung nach Anspruch 7 oder 8, worin T und T' beide O sind, R H oder (C₁-C₆)-Alkyl ist und R_{1c} und R_{3c} unabhängig voneinander (C₁-C₆)-Alkyl oder Phenyl sind, wobei das Phenyl gegebenenfalls mit (C₁-C₆)-Alkyl, Hydroxy-(C₁-C₆)-alkyl, Amino-(C₁-C₆)-alkyl, Halogen, Carboxyl, CO₂-(C₁-C₆)-alkyl, CONH₂, COH, CO-(C₁-C₆)-alkyl, SO₃H, SO₂NH₂, Phenoxy, (C₁-C₆)-Alkylthio, NH₂ oder (C₁-C₆)-Alkoxy substituiert ist.

10. Verbindung nach Anspruch 9, worin R_{1c} und R_{3c} unabhängig voneinander Phenyl sind, wobei das Phenyl gegebenenfalls mit (C₁-C₆)-Alkyl, Hydroxy-(C₁-C₆)-alkyl, Amino-(C₁-C₆)-alkyl, Halogen, Carboxyl, CO₂-(C₁-C₆)-alkyl, CONH₂, COH, CO-(C₁-C₆)-alkyl, SO₃H, SO₂NH₂, Phenoxy, (C₁-C₆)-Alkylthio, NH₂ oder (C₁-C₆)-Alkoxy substituiert ist.

11. Verbindung nach Anspruch 9, worin R H ist und R_{1c} und R_{3c} unabhängig voneinander (C₁-C₆)-Alkyl oder Phenyl sind.

12. Verbindung nach einem der Ansprüche 1 bis 9, worin Y und Y' beide O sind.

13. Ruthenium(II)-Verbindung der Formel (I): 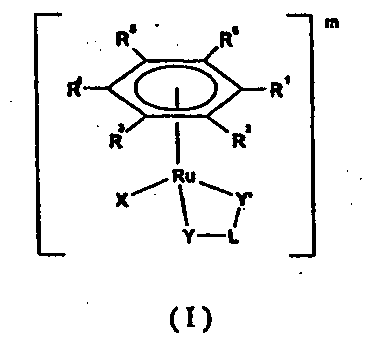 zur Verwendung in einer Therapie, worin:
R¹, R², R³, R⁴, R⁵ und R⁶ unabhängig voneinander für H, (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Halogen, CO₂R⁷, CONR⁸R⁹, COR¹⁰, SO₃H, SO₂NR¹¹R¹², Aryloxy, (C₁-C₆)-Alkoxy, (C₁-C₆)-Alkylthio, -N=N-R¹³, NR¹⁴R¹⁵, Aryl oder Aralkyl stehen, wobei die letzteren beiden Gruppen gegebenenfalls am aromatischen Ring mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7a}, CONR^{8a}R^{9a}, COR^{10a}, SO₃G, SO₂NR^{11a}R^{12a}, Acyloxy, (C₁-C₆)-Alkoxy, (C₁-C₆)-Alkylthio, -N=N-R^{13a}, NR^{14a}R^{15a} ausgewählten Gruppen substituiert sind, oder R¹ und R² gemeinsam mit dem Ring, an den sie gebunden sind, eine gesättigte oder ungesättigte carbozyklische oder heterozyklische Gruppe mit bis zu drei 3- bis 8-gliedrigen carbozyklischen oder heterozyklischen Ringen darstellen, worin jeder carbozyklische oder heterozyklische Ring gegebenenfalls an einen oder mehrere andere carbozyklische oder heterozyklische Ringe kondensiert ist und worin jeder der Ringe gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b}, CONR^{8b}R^{9b}, COR^{10b}, SO₃G', SO₂NR^{11b}R^{12b}, Aryloxy, (C₁-C₆)-Alkylthio, -N=N-R^{13b}, NR^{14b}R^{15b} oder (C₁-C₆)-Alkoxy ausgewählten Gruppen substituiert ist;
R⁷, R⁸, R⁹, R¹⁰, R¹¹, R¹², R¹³, R¹⁴, R¹⁵, R^{7a}, R^{8a}, R^{9a}, R^{10a}, R^{11a}, R^{12a}, R^{13a}, R^{14a}, R^{15a}, R^{7b}, R^{8b}, R^{9b}, R^{10b}, R^{11b}, R^{12b}, R^{13b}, R^{14b} und R^{15b} unabhängig voneinander aus H, (C₁-C₆)-Alkyl, Aryl oder Aralkyl ausgewählt sind;
X ein neutraler oder negativ geladener O-, N- oder S-Donorligand oder ein Halogen ist;
G und G' unabhängig voneinander aus Alkalimetallen, Aryl, Aralkyl und (C₁-C₆)-Alkyl ausgewählt sind;
Y-L-Y' ein zweizähniger Ligand ist, der eine negative Ladung trägt, wobei ein Teil der Ladung auf sowohl Y als auch Y' vorliegt, Y und Y' unabhängig voneinander aus O, S oder NR¹⁶ ausgewählt sind, worin R¹⁶ H, (C₁-C₆)-Alkyl, Aryl oder Aralkyl ist, und L eine Gruppe ist, die Y und Y' verbindet und eine oder mehrere aus (C₁-C₆)-Alkylen, (C₁-C₆)-Alkenylen, (C₁-C₆)-Alkinylen, Arylen, Aralkylen, Alkarylen, wobei jede der letzteren sechs Gruppen gegebenenfalls substituiert ist, Ferrocenylen, Se, Se-Se, S-S, N=N und C=O ausgewählte Gruppen umfasst;
m = -1, 0 oder +1 ist und die Verbindung ein Gegenion umfasst, wenn m -1 oder +1 ist;
wobei die Verbindung der Formel (I) gegebenenfalls in Form eines Dimers vorliegt, worin zwei L-Gruppen entweder direkt oder durch eine Gruppe verbunden sind, die eines oder mehrere aus (C₁-C₆)-Alkylen, (C₁-C₆)-Alkenylen, Arylen, Aralkylen, Alkarylen, Se, Se-Se, S-S, N=N und C=O umfasst, oder worin L zwei Y-Gruppen und zwei Y'-Gruppen trägt.

14. Verbindung nach Anspruch 13, worin R¹, R², R³, R⁴, R⁵ und R⁶ unabhängig voneinander aus H, (C₁-C₆)-Alkyl und Phenyl ausgewählt sind oder R¹ und R² gemeinsam mit dem Ring, an den sie gebunden sind, für Anthracen oder ein hydriertes Derivat von Anthracen stehen, wobei das Phenyl und Anthracen oder hydrierte Derivat der Anthracengruppe gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Phenyl, Benzyl, Halogen, Carboxyl, CO₂-(C₁-C₆)-alkyl, CONH₂, COH, CO-(C₁-C₆)-alkyl, SO₃H, SO₂NH₂, Phenoxy, (C₁-C₆)-Alkylthio, NH₂ oder (C₁-C₆)-Alkoxy ausgewählten Gruppen substituiert sind.

15. Verbindung nach Anspruch 13 oder Anspruch 14, worin m = 0 ist.

16. Verbindung nach einem der Ansprüche 13 bis 15, worin X ein Halogen oder CH₃CN ist.

17. Verbindung nach einem der Ansprüche 13 bis 16, worin Y-L-Y' aus Liganden der Formeln (II) bis (X) ausgewählt ist: 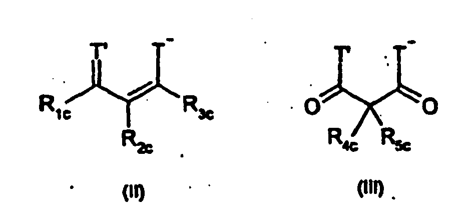 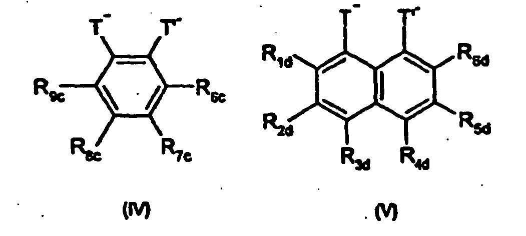 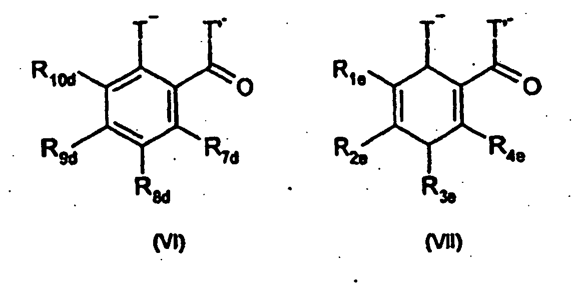 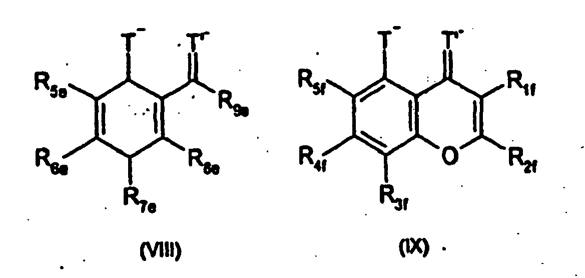 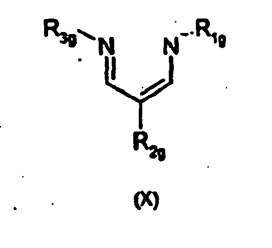 worin T und T' unabhängig voneinander aus O und S ausgewählt sind,
R_{1g} und R_{3g} unabhängig voneinander H, (C₁-C₆)-Alkyl, Aryl oder Aralkyl sind,
R_{1c} bis R_{5f} und R_{2g} unabhängig voneinander H, (C₁-C₆)-Alkyl, Aryl, Aralkyl sind, worin die letzteren beiden Gruppen und die entsprechenden Gruppen für R_{1g} und R_{3g} gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b}, CONR^{8b}R^{9b}, COR^{10b}, SO₃G', SO₂NR^{11b}R^{12b}, Aryloxy, (C₁-C₆)-Alkylthio, -N=N-R^{13b}, NR^{14b}R^{15b} oder (C₁-C₆)-Alkoxy, worin R^{7b}, R^{8b}, R^{9b}, R^{10b}, R^{11b}, R^{12b}, R^{13b}, R^{14b} und R^{15b} wie in Anspruch 13 definiert sind, ausgewählten Gruppen substituiert sind.

18. Verbindung nach einem der Ansprüche 13 bis 16, worin Y-L-Y' ausgewählt ist aus: 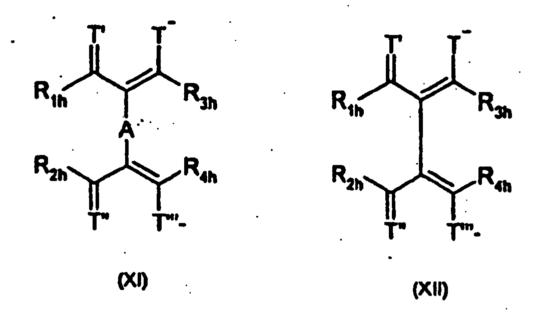 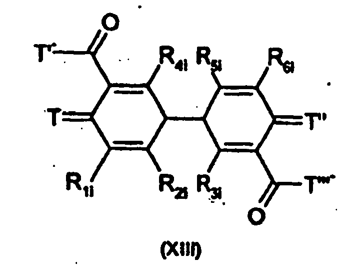 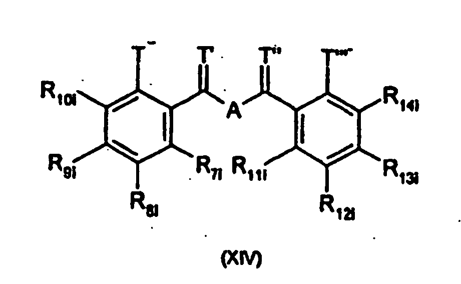 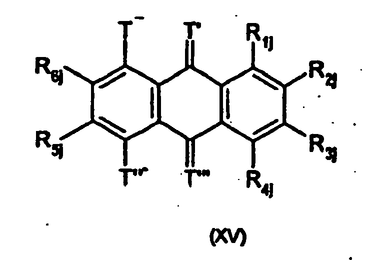 worin T, T', T" und T''' unabhängig voneinander aus O und S ausgewählt sind,
A eine oder mehrere aus (C₁-C₆)-Alkylen, (C₁-C₆)-Alkenylen, (C₁-C₆)-Alkinylen, Arylen, Aralkylen, Alkarylen, Ferrocenylen, Se, Se-Se, S-S, N=N und C=O ausgewählte Gruppen umfasst
und R₁ₕ bis R₆ⱼ unabhängig voneinander H, (C₁-C₆)-Alkyl, Aryl, Aralkyl sind, worin die letzteren beiden Gruppen gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b}, CONR^{8b}R^{9b}, COR^{10b}, SO₃G', SO₂NR^{11b}R^{12b}, Acyloxy, (C₁-C₆)-Alkylthio, -N=N-R^{13b}, NR^{14b}R^{15b} oder (C₁-C₆)-Alkoxy ausgewählten Gruppen substituiert sind, worin R^{7b}, R^{8b}, R^{9b}, R^{10b}, R^{11b}, R^{12b}, R^{13b}, R^{14b} und R^{15b} wie in Anspruch 13 definiert sind.

19. Verbindung nach einem der Ansprüche 13 bis 16, worin Y-L-Y' 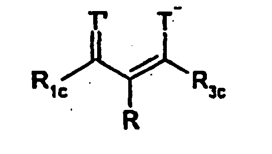 ist,
worin T und T' unabhängig voneinander O und S sind und R, R_{1c} und R_{3c} unabhängig voneinander H, (C₁-C₆)-Alkyl, Aryl, Aralkyl sind, worin die letzteren beiden Gruppen gegebenenfalls mit einer oder mehreren unabhängig voneinander aus (C₁-C₆)-Alkyl, (C₂-C₆)-Alkenyl, (C₂-C₆)-Alkinyl, Hydroxy-(C₁-C₆)-alkyl, Amino-(C₁-C₆)-alkyl, Aryl, Aralkyl, Halogen, CO₂R^{7b}, CONR^{8b}R^{9b}, COR^{10b}, SO₃G', SO₂NR^{11b}R^{12b}, Aryloxy, (C₁-C₆)-Alkylthio, -N=N-R^{13b}, NR^{14b}R^{15b} oder (C₁-C₆)-Alkoxy ausgewählten Gruppen substituiert sind, worin R^{7b}, R^{8b}, R^{9b}, R^{10b}, R^{11b}, R^{12b}, R^{13b}, R^{14b} und R^{15b} wie in Anspruch 13 definiert sind.

20. Verbindung nach Anspruch 19, worin T und T' beide O sind, R H oder (C₁-C₆)-Alkyl ist und R_{1c} und R_{3c} unabhängig voneinander (C₁-C₆)-Alkyl oder Phenyl sind, wobei das Phenyl gegebenenfalls mit (C₁-C₆)-Alkyl, Hydroxy-(C₁-C₆)-alkyl, Amino-(C₁-C₆)-alkyl, Halogen, Carboxyl, CO₂-(C₁-C₆)-alkyl, CONH₂, COH, CO-(C₁-C₆)-alkyl, SO₃H, SO₂NH₂, Phenoxy, (C₁-C₆)-Alkylthio, NH₂ oder (C₁-C₆)-Alkoxy substituiert ist.

21. Verbindung nach Anspruch 20, worin R H ist und R_{1c} und R_{3c} unabhängig voneinander (C₁-C₆)-Alkyl oder Phenyl sind.

22. Verbindung nach einem der Ansprüche 13 bis 21, worin Y und Y' beide O sind.

23. Verwendung einer Verbindung der Formel (I) nach einem der Ansprüche 13 bis 22 zur Herstellung eines Medikaments zur Behandlung und/oder Prävention von Krebs.

24. Pharmazeutische Zusammensetzung, die eine Verbindung der Formel (I) nach einem der Ansprüche 13 bis 21 gemeinsam mit einem oder mehreren pharmazeutisch annehmbaren Arzneimittelträgern umfasst.

25. Verbindung der Formel (I) nach einem der Ansprüche 13 bis 21 oder eine Zusammensetzung nach Anspruch 24 zur Verwendung bei der Behandlung und/oder Prävention von Krebs.

26. Verfahren zur Herstellung einer Verbindung nach einem der Ansprüche 1 bis 12, welches das Umsetzen einer Verbindung der Formel [(η⁶-C₆(R¹)(R²)(R³)(R⁴)(R⁵)(R⁶))RuX₂], gegebenenfalls in Form eines Dimers, mit Y-L-Y in einem für die Reaktion geeigneten Lösungsmittel umfasst, worin R¹, R², R³, R⁴, R⁵, R⁶, X, Y, Y' und L wie in Anspruch 1 definiert sind.

## Revendications

1. Composé de ruthénium (II) de la formule (I) : 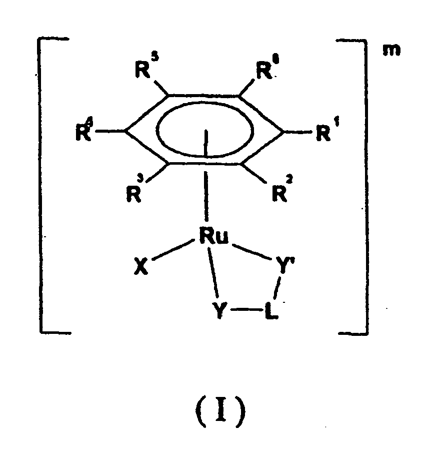 où R¹, R², R³, R⁴, R⁵ et R⁶ représentent indépendamment H, alkyle (C₁-C₆), alcényle (C₂-C₆), alkynyle (C₁-C₆), hydroxyalkyle (C₁-C₆), aminoalkyle (C₁-C₆), halo, CO₂R⁷, CONR⁸R⁹, COR¹⁰, SO₃H, SO₂NR¹¹R¹², aryloxy, alcoxy (C₁-C₆), alkylthio (C₁-C₆), -N=N-R¹³, NR¹⁴R¹⁵, aryle ou aralkyle, ces deux derniers groupes étant facultativement substitués sur le cycle aromatique par un ou plusieurs groupes indépendamment sélectionnés parmi alkyle (C₁-C₆), alcényle (C₂-C₆), alkynyle (C₂-C₆), hydroxyalkyle (C₁-C₆), aminoalkyle (C₁-C₆), aryle, aralkyle, halo, CO₂R^{7a}, CONR^{8a}R^{9a}, COR^{10a}, SO₃G, SO₂NR^{11a}R^{12a}, aryloxy, alcoxy (C₁-C₆), alkylthio (C₁-C₆), -N=N-R¹³, NR¹⁴R¹⁵ ou bien R¹ et R² avec le cycle auquel ils sont liés représentent un groupe carbocyclique ou hétérocyclique saturé ou insaturé contenant jusqu'à 3 cycles carbocycliques ou hétérocycliques de 3 à 8 membres, où chaque cycle carbocyclique ou hétérocyclique peut être fusionné à un ou plusieurs autres cycles carbocycliques et où chacun des cycles peut être facultativement substitué par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), aryle, aralkyle, halo, CO₂R^{7b}, CONR^{8b}R^{9b}, COR^{10b}, SO₃G', SO₂NR^{11b}R^{12b}, aryloxy, alkylthio(C₁-C₆) , -N=N-R¹³, NR^{14b}R^{15b} ou alcoxy(C₁-C₆) ;
R⁷, R⁸, R⁹, R¹⁰, R¹¹, R¹², R¹³, R¹⁴, R¹⁵, R^{7a}, R^{8a}, R^{8a}, R^{9a}, R^{10a}, R^{11a}, R^{12a}, R^{13a}, R^{14a}, R^{15a}, R^{7b}, R^{8b}, R^{9b}, R^{10b}, R^{11b}, R^{12b}, R^{13b}, R^{14b} et R^{15b}, sont indépendamment sélectionnés parmi H, alkyle(C₁-C₆), aryle ou aralkyle ;
X est un ligand donneur de O, N ou S neutre ou négativement chargé ou halo ;
G et G' sont indépendamment sélectionnés parmi des métaux alcalins, aryle, aralkyle et alkyle(C₁-C₆) ;
Y-L-Y' est un ligand bidenté portant une charge négative avec une proportion de la charge sur Y et Y', Y et Y' sont indépendamment sélectionnés parmi O, S ou NR¹⁶, où R¹⁶ est H, alkyle(C₁-C₆), aryle ou aralkyle et L est un groupe enchaînant Y et Y' et comprend un ou plusieurs groupes sélectionnés parmi alkylène (C₁-C₆), alcénylène (C₁-C₆), alkynylène(C₁-C₆), arylène, aralkylène, alkarylène, chacun de ces six derniers groupes étant facultativement substitué, ferrocénylène, Se, Se-Se, S-S, N=N et C=O ;
m est -1, 0 ou +1 et le composé comprend un contre-ion où m est -1 ou +1 ;
le composé de formule (I) étant facultativement sous la forme d'un dimère dans lequel deux groupes L sont enchaînés soit directement ou par un groupe comprenant un ou plusieurs de alkylène(C₁-C₆), alcénylène(C₁-C₆), arylène, aralkylène, alcarylène, Se, Se-Se, S-S, N=N et C=O ou dans lequel L porte deux groupes Y et deux groupes Y' ;
aux conditions que :
quand Y-L-Y' est (CH₃C(O)CHC(O)CH₃)⁻, X est halo ou un ligand donneur de N, R¹, R², R³, R⁴, R⁵ et R⁶ avec le cycle auquel ils sont liés ne représentent pas 4-isopropyl-1-méthylbenzène ;
quand Y-L-Y' est (CH₃C(O)CHC(O)CH₃)⁻ et X est chloro, (CH₃)₂SO, CH₃CN, pyridine ou (CH₃C(O)CHC(O)CH₃)⁻ : R¹, R², R³, R⁴, R⁵ et R⁶ ne soient pas tous H ou tous méthyle ; R¹, R³ et
R⁵ ne soient pas tous H quand R², R⁴ et R⁶ sont tous méthyle ; et R², R⁴ et R⁶ ne soient pas tous H quand R¹, R³ et R⁵ sont tous méthyle ;
quand Y-L-Y' est (CF₃C(O)CHC(O)CHF₃)⁻ et que X est chloro, R¹, R², R³, R⁴, R⁵ et R⁶ ne soient pas tous H ni tous méthyle ; R¹, R³ et R⁵ ne soient pas tous H quand R², R⁴ et R⁶ sont tous méthyle ; et R², R⁴ et R⁶ ne soient pas tous H quand R¹, R³ et R⁵ sont tous méthyle ;
quand Y-L-Y' est (CF₃C(O)CHC(CO)OEt)⁻ et X est chloro, R¹, R², R³, R⁴, R⁵ et R⁶ avec le cycle auquel ils sont liés ne représentent pas 4-isopropyl-1-méthylbenzène ;
quand Y-L-Y' est ((ferrocénylène)C(O)CHC(O)(ferrocénylène))⁻ et X est chloro, R¹, R², R³, R⁴, R⁵ et R⁶ avec le cycle auquel ils sont liés ne représentent pas 4-isopropyl-1-méthylbenzène ;
quand R¹, R², R³, R⁴, R⁵ et R⁶ avec le cycle auquel ils sont liés représentent 4-isopropyl-1-méthylbenzène et que X est chloro, Y-L-Y' ne soit ni (3-OCH₃-4-OH-PhCHCHC(O)CHC(O)CHCH-3-OCH₃-4-OH-Ph)⁻, (3-OCH₃-4-OCH₃-PhCHCHC(O)C(CH₃)C(O)CHCH-3-OCH₃-4-OCH₃-Ph)⁻, (3-OCH₃-4-COOCH₃-PhCHCHC(O) CHC(O) CHCH-3-OCH₃-4-COOCH₃-Ph)⁻, (4-OH-PhCHCHC(O)CHC(O)CHCH-4-OH-Ph)⁻ ni (3-OCH₃-4-OCH₃-PhCHCHC(O)CHC(O)CHCH-3-OCH₃-4-OCH₃-Ph)⁻.

2. Composé selon la revendication 1, où R¹, R², R³, R⁴, R⁵ et R⁶ sont indépendamment sélectionnés parmi H, alkyle(C₁-C₆) et phényle ou bien R¹ et R² avec le cycle auquel ils sont liés représentent anthracène, ou un dérivé hydrogéné de l'anthracène, ledit phényle et ledit anthracène ou un dérivé hydrogéné d'un groupe anthracène étant facultativement substitués par un ou plusieurs groupes indépendamment sélectionnés parmi alkyle (C₁-C₆), alcényle (C₂-C₆), alkynyle (C₂-C₆), hydroxyalkyle (C₁-C₆), aminoalkyle (C₁-C₆) phényle, benzyle, halo, carboxyle, CO₂alkyle (C₁-C₆), CONH₂, COH, COalkyle(C₁-C₆), SO₃H, SO₂NH₂, phénoxy, alkylthio(C₁-C₆), NH₂ ou alcoxy(C₁-C₆).

3. Composé selon la revendication 1 ou 2, où m est 0.

4. Composé selon l'une quelconque des revendications 1 à 3, où X est halo ou CH₃CN.

5. Composé selon l'une quelconque des revendications 1 à 4, où Y-L-Y' est sélectionné parmi des ligands des formules (II) à (X) : 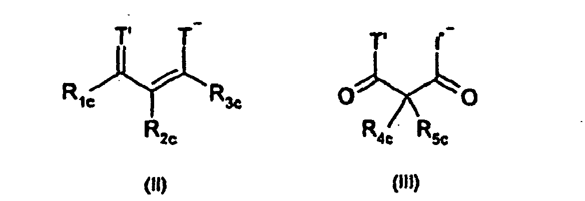 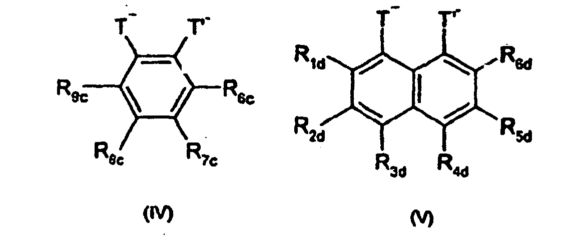 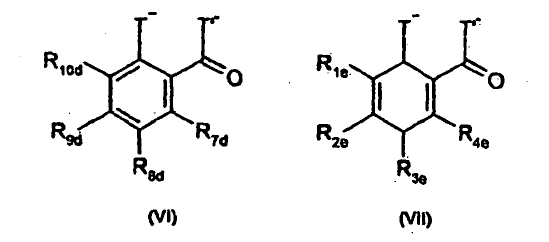 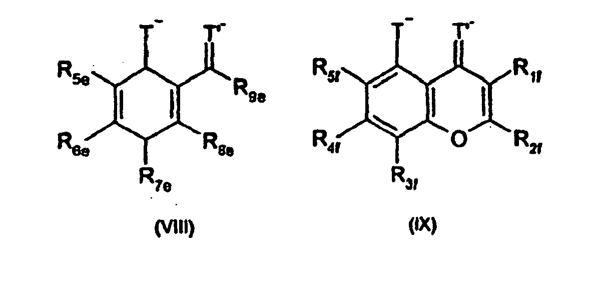 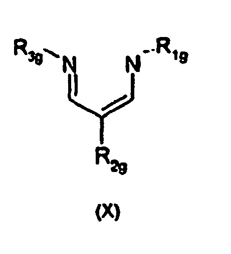 où T et T' sont indépendamment sélectionnés parmi O et S,
R_{1g} et R_{3g} sont indépendamment H, alkyle(C₁-C₆), aryle ou alkyle,
R_{1c} à R_{5f} et R_{2g} sont indépendamment H, alkyle(C₁-C₆), aryle, aralkyle, où ces deux derniers groupes et les groupes correspondant pour R_{1g} et R_{3g} sont facultativement substitués par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), , aryle, aralkyle, halo, CO₂R⁷, CONR^{8b}R^{9b}, COR^{10b}, SO₃G', SO₂G', SO₂NR^{11b}R^{12b}, aryloxy, alkylthio(C₁-C₆), -N=N-R^{13b}, NR^{14b}R^{15b} ou alcoxy(C₁-C₆) ; ou R^{7b}, R^{8b}, R^{9b}, R^{10b}, R^{11b}, R^{12b}, R^{13b}, R^{14b}, et R^{15b} sont tels que définis à la revendication 1.

6. Composé selon l'une quelconque des revendications 1 à 4, où Y-L-Y' est sélectionné parmi : 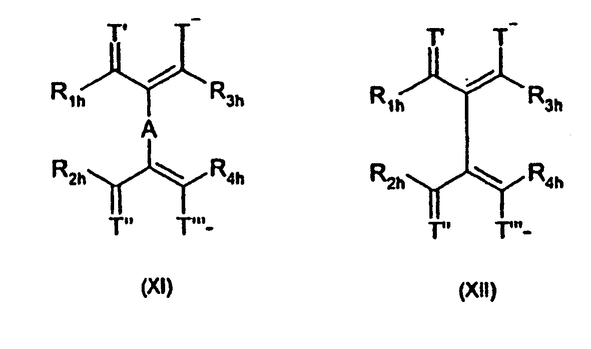 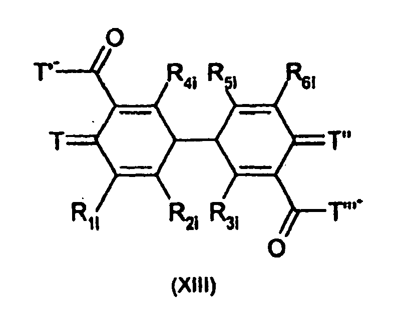 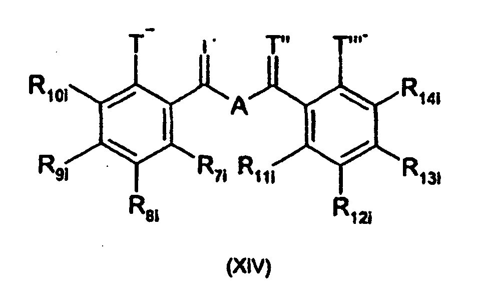 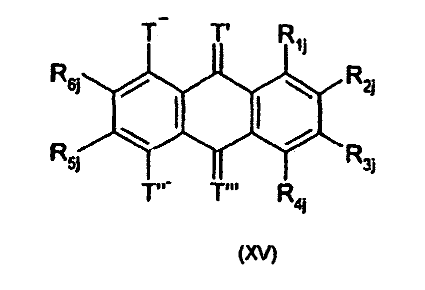 où T, T', T" et T''' sont indépendamment sélectionnés parmi O et S,
A comprend un ou plusieurs groupes sélectionnés parmi alkylène (C₁-C₆), alcénylène (C₁-C₆), alkylynène (C₁-C₆), arylène, aralkylène, alcarylène, ferrocénylène, Se, Se-Se, S-S, N=N et C=O
et R₁ₕ et R₆ⱼ sont indépendamment H, alkyle(C₁-C₆), aryle, aralkyle, où ces deux derniers groupes sont facultativement substitués par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), aryle, aralkyle, halo, CO₂R^{7b}, CONR^{8b}R^{9b}, COR^{10b}, SO₃G', SO₂NR^{11b}R^{12b}, aryloxy, alkylthio (C₁-C₆), -N=N-R^{13b}, NR^{14b}R^{15b} ou alcoxy(C₁-C₆), où R^{7b}, R^{8b}, R^{9b}, R^{10b}, R^{11b}, R^{12b}, R^{13b}, R^{14b} et R^{15b} sont tels que définis à la revendication 1.

7. Composé selon l'une quelconque des revendications 1 à 4, où Y-L-Y' est : 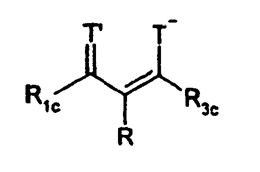 où T et T' sont indépendamment O et S, et R₁, R_{1c} et R_{3c} sont indépendamment H, alkyle(C₁-C₆), aryle ou aralkyle, où ces deux derniers groupes sont facultativement substitués par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), aryle, aralkyle, halo, CO₂R^{7b}, CONR^{8b}R^{9b}, COR^{10b}, SO₃G', SO₂NR^{11b}R^{12b}, aryloxy, alkylthio(C₁-C₆), -N=N-R^{13b}, NR^{14b}R^{15b} ou alcoxy(C₁-C₆) ; où R^{7b}, R^{8b}, R^{9b}, R^{10b}, R^{11b}, R^{12b}, R^{13b}, R^{14b} et R^{15b} sont tels que définis à la revendication 1.

8. Composé selon la revendication 7, où l'un de R¹, R², R³, R⁴, R⁶ est phényle et les autres groupes sont H.

9. Composé selon la revendication 7 ou la revendication 8, où T et T' sont tous deux O, R et H ou alkyle(C₁-C₆), et R_{1c} et R_{3c} sont indépendamment alkyle(C₁-C₆) ou phényle, ledit phényle facultativement substitué par alkyle(C₁-C₆), hydroxyalkyle(C₁-C₆), aminoalkyle(C₁-C₆), halo, carboxyle, CO₂alkyle(C₁-C₆), CONH₂, COH, COalkyle(C₁-C₆), SO₃H, SO₂NH₂, phénoxy, alkylthio(C₁-C₆), NH₂ ou alcoxy(C₁-C₆) .

10. Composé selon la revendication 9, où R_{1c} et R_{3c} sont indépendamment phényle, ledit phényle facultativement substitué par alkyle(C₁-C₆), hydroxyalkyle(C₁-C₆), aminoalkyle(C₁-C₆), halo, carboxyle, CO₂alkyle(C₁-C₆), CONH₂, COH, COalkyle(C₁-C₆), SO₃H, SO₂NH₂, phénoxy, alkylthio(C₁-C₆), NH₂ ou alcoxy(C₁-C₆).

11. Composé selon la revendication 9, où R est H et R_{1c} et R_{3c} sont indépendamment alkyle(C₁-C₆) ou phényle.

12. Composé selon l'une quelconque des revendications 1 à 9, où Y est Y' sont tous deux O.

13. Composé de ruthénium (II) de formule (I) : 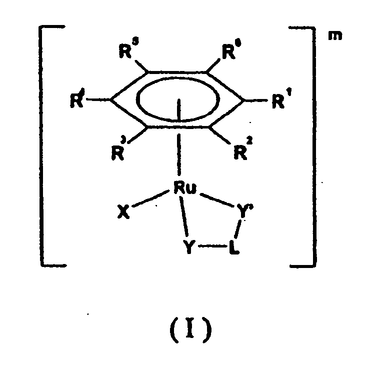 pour une utilisation en thérapie :
où R¹, R², R³, R⁴, R⁵ et R⁶ représentent indépendamment H, alkyle(C₁-C₆), alcényle(C₂-C₆), alkynyle(C₁-C₆), hydroxyalkyle(C₁-C₆), aminoalkyle(C₁-C₆), halo, CO₂R⁷, CONR⁸R⁹, COR¹⁰, SO₃H, SO₂NR¹¹R¹², aryloxy, alcoxy(C₁-C₆), alkylthio(C₁-C₆), -N=N-R¹³, NR¹⁴R¹⁵, aryle ou aralkyle, ces deux derniers groupes étant facultativement substitués sur le cycle aromatique par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), aryle, aralkyle, halo, CO₂R^{7a}, CONR^{8a}R^{9a}, COR^{10a}, SO₃G, SO₂NR^{11a}R^{12a}, aryloxy, alcoxy(C₁-C₆), alkylthio(C₁-C₆), -N=N-R^{13a}, NR^{14a}R^{15a} ou bien R¹ et R² avec le cycle auquel ils sont liés représentent un groupe carbocyclique ou hétérocyclique saturé ou insaturé contenant jusqu'à 3 cycles carbocycliques ou hétérocycliques de 3 à 8 membres, où chaque cycle carbocyclique ou hétérocyclique peut être fusionné à un ou plusieurs autres cycles carbocycliques ou hétérocycliques et où chacun des cycles peut être facultativement substitué par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), aryle, aralkyle, halo, CO₂R^{7b}, CONR^{8b}R^{9b}, COR^{10b}, SO₃G', SO₂NR^{11b}R^{12b}, aryloxy, alkylthio(C₁-C₆), -N=N-R¹³, NR^{14b}R^{15b} ou alcoxy(C₁-C₆) ;
R⁷, R⁸, R⁹, R¹⁰, R¹¹, R¹², R¹³, R¹⁴, R¹⁵, R^{7a}, R^{8a}, R^{9a}, R^{10a}, R^{11a}, R^{12a}, R^{13a}, R^{14a}, R^{15a}, R^{7b}, R^{8b}, R^{9b}, R^{10b}, R^{11b}, R^{12b}, R^{13b}, R^{14b} et R^{15b}, sont indépendamment sélectionnés parmi H, alkyle(C₁-C₆), aryle ou aralkyle ;
X est un ligand donneur de O, N ou S neutre, négativement chargé ou bien halo ;
G et G' sont indépendamment sélectionnés parmi des métaux alcalins, aryle, aralkyle et alkyle(C₁-C₆) ;
Y-L-Y' est un ligand bidenté portant une charge négative avec une proportion de la charge sur Y et Y', Y et Y' sont indépendamment sélectionnés parmi O, S ou NR¹⁶, où R¹⁶ est H, alkyle(C₁-C₆), aryle ou aralkyle et L est un groupe enchaînant Y et Y' et comprend un ou plusieurs groupes sélectionnés parmi alkylène(C₁-C₆), alcénylène(C₁-C₆), alkynylène(C₁-C₆), aralkylène, chacun de ces six derniers groupes étant facultativement substitué, ferrocénylène, Se, Se-Se, S-S, N=N et C=O ;
m est -1, 0 ou +1 et le composé comprend un contre-ion où m est -1 ou +1 ;
le composé de formule (I) étant facultativement sous la forme d'un dimère dans lequel deux groupes L sont enchaînés soit directement ou par un groupe comprenant un ou plusieurs alkylène(C₁-C₆), alcénylène(C₁-C₆), arylène, aralkylène, alcarylène, Se, Se-Se, S-S, N=N et C=O ou dans lequel L porte deux groupes Y et deux groupes Y'.

14. Composé selon la revendication 13, où R¹, R², R³, R⁴, R⁵ et R⁶ sont indépendamment sélectionnés parmi H, alkyle (C₁-C₆) et phényle ou bien R¹ et R² avec le cycle auquel ils sont liés représentent anthracène, ou un dérivé hydrogéné de l'anthracène, ledit phényle et ledit anthracène ou un dérivé hydrogéné d'un groupe anthracène étant facultativement substitués par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), phényle, benzyle, halo, carboxyle, CO₂alkyle(C₁-C₆), CONH₂, COH, COalkyle(C₁-C₆), SO₃H, SO₂NH₂, phénoxy, alkylthio(C₁-C₆), NH₂ ou alcoxy(C₁-C₆) .

15. Composé selon la revendication 13 ou la revendication 14, où m est 0.

16. Composé selon l'une quelconque des revendications 13 à 15, où X est halo ou CH₃CN.

17. Composé selon l'une quelconque des revendications 13 à 16, où Y-L-Y' est sélectionné parmi des ligands des formules (II) à (X) : 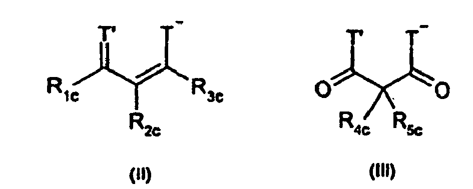 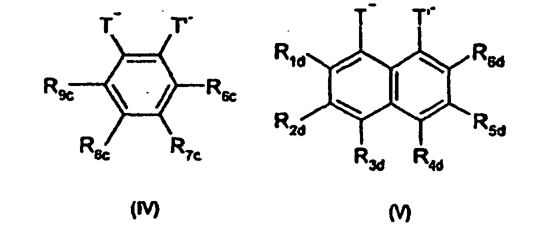 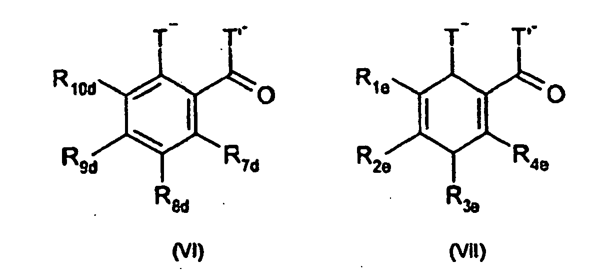 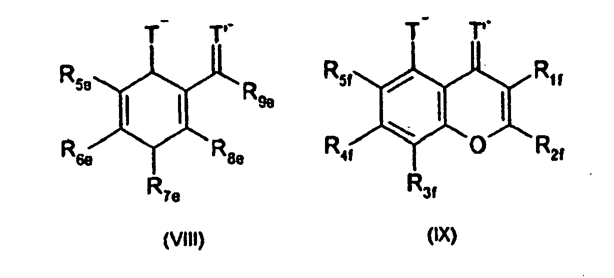 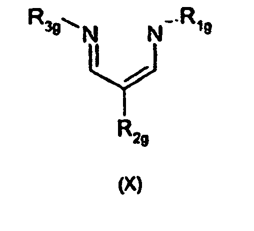 où T et T' sont indépendamment sélectionnés parmi O et S,
R_{1g} et R_{3g} sont indépendamment H, alkyle(C₁-C₆), aryle ou aralkyle,
R_{1c} à R_{5f} et R_{2g} sont indépendamment H, alkyle(C₁-C₆), aryle, aralkyle, où ces deux derniers groupes et les groupes correspondants pour R_{1g} et R_{3g} sont facultativement substitués par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), , aryle, aralkyle, halo, CO₂R⁷, CONR^{8b}R^{9b}, COR^{10b}, SO₃G', SO₂G', SO₂NR^{11b}R^{12b}, aryloxy, alkylthio(C₁-C₆), -N=N-R^{13b}, NR^{14b}R^{15b} ou alcoxy(C₁-C₆) ; où R^{7b}, R^{8b}, R^{9b}, R^{10b}, R^{11b}, R^{12b}, R^{13b}, R^{14b} et R^{15b} sont tels que définis à la revendication 13.

18. Composé selon l'une quelconque des revendications 13 à 16, où Y-L-Y' est sélectionné parmi : 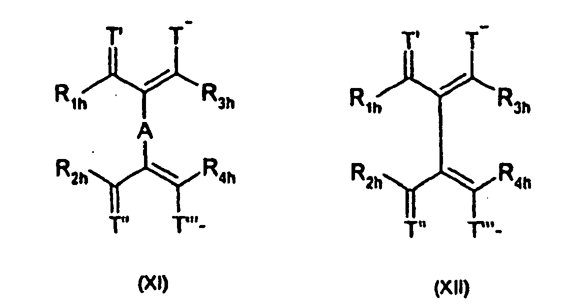 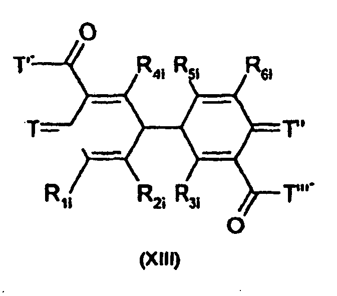 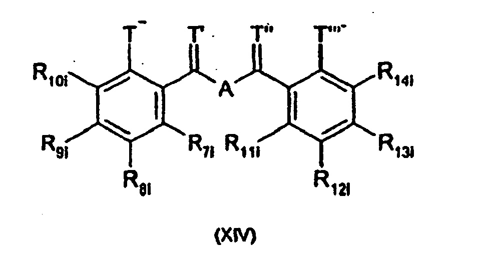 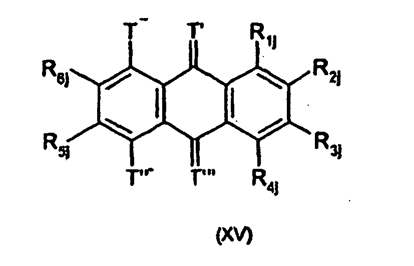 où T, T', T" et T''' sont indépendamment sélectionnés parmi O et S,
A comprend un ou plusieurs groupes sélectionnés parmi alkylène(C₁-C₆), alcénylène(C₁-C₆), alkynylène(C₁-C₆), arylène, aralkylène, alcarylène, ferrocénylène, Se, Se-Se, S-S, N=N et C=O
et R₁ₕ et R₆ⱼ sont indépendamment H, alkyle(C₁-C₆), aryle, aralkyle, où ces deux derniers groupes sont facultativement substitués par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), aryle, aralkyle, halo, CO₂R^{7b}, CONR^{8b}R^{9b}, COR^{10b}, SO₃G', SO₂NR^{11b}R^{12b}, aryloxy, alkylthio(C₁-C₆), -N=N-R^{13b}, NR^{14b}R^{15b} ou alcoxy(C₁-C₆) ; où R^{7b}, R^{8b}, R^{9b}, R^{10b}, R^{11b}, R^{12b}, R^{13b}, R^{14b} et R^{15b} sont tels que définis à la revendication 13.

19. Composé selon l'une quelconque des revendications 13 à 16, où Y-L-Y' est : 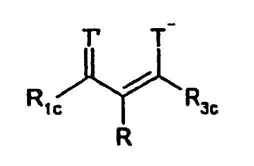 où T et T' sont indépendamment O et S, et R₁, R_{1c} et R_{3c} sont indépendamment H, alkyle(C₁-C₆), aryle ou aralkyle, où ces deux derniers groupes sont facultativement substitués par un ou plusieurs groupes indépendamment sélectionnés parmi alkyle(C₁-C₆), alcényle(C₂-C₆), alkynyle(C₂-C₆), hydroxyalkyle(C₁-C₆), aminoalkyle(C₁-C₆), aryle, aralkyle, halo, CO₂R^{7b}, CONR^{8b}R^{9b}, COR^{10b}, SO₃G', SO₂NR^{11b}R^{12b}, aryloxy, alkylthio(C₁-C₆), -N=N-R^{13b}, NR^{14b}R^{15b} ou alcoxy(C₁-C₆) ; où R^{7b}, R^{8b}, R^{9b}, R^{10b}, R^{11b}, R^{12b}, R^{13b}, R^{14b} et R^{15b} sont tels que définis à la revendication 13.

20. Composé selon la revendication 19, où T et T' sont tous deux O, R est H ou alkyle(C₁-C₆), et R_{1c} et R_{3c} sont indépendamment alkyle(C₁-C₆) ou phényle, ledit phényle facultativement substitué par alkyle(C₁-C₆), hydroxyalkyle(C₁-C₆), aminoalkyle(C₁-C₆), halo, carboxyle, CO₂alkyle(C₁-C₆), CONH₂, COH, COalkyle(C₁-C₆), SO₃H, SO₂NH₂, phénoxy, alkylthio(C₁-C₆), NH₂ ou alcoxy(C₁-C₆).

21. Composé selon la revendication 20, où R est H et R_{1c} et R_{3c} sont indépendamment alkyle(C₁-C₆) ou phényle.

22. Composé selon l'une quelconque des revendications 13 à 21 où Y et Y' sont tous deux O.

23. Utilisation d'un composé de formule (I) selon l'une quelconque des revendications 13 à 22 dans la fabrication d'un médicament pour le traitement et/ou la prévention du cancer.

24. Composition pharmaceutique comprenant un composé de formule (I) selon l'une quelconque des revendications 13 à 21 avec un ou plusieurs excipients pharmaceutiquement acceptables.

25. Composé de formule (I) selon l'une quelconque des revendications 13 à 21 ou composition de la revendication 24 à utiliser dans le traitement et/ou la prévention du cancer.

26. Procédé pour la préparation du composé de l'une quelconque des revendications 1 à 12 qui comprend la réaction d'un composé de formule [(η⁶-C₆(R¹)(R²)(R³)(R⁴)(R⁵)(R⁶))RuX₂, optionnellement sous la forme d'un dimère, avec Y-L-Y, dans un solvant approprié pour la réaction où R¹, R², R³, R⁴, R⁵, R⁶, X, Y, Y' et L sont tels que définis dans la revendication 1.
